# EUROPÄISCHE PATENTSCHRIFT

(11) **EP 1 543 032 B1**
(45) Veröffentlichungstag und Bekanntmachung des Hinweises auf die Patenterteilung: **07.01.2009**
(21) Anmeldenummer: 03769315.7
(22) Anmeldetag: 24.09.2003
(51) Int. Cl.: C07K 14/705, C12N 15/12, A61K 38/17

(54) **VERFAHREN ZUR RATIONALEN MUTAGENESE VON A/BETA T-ZELL REZEPTOREN UND ENTSPRECHEND MUTIERTE MDM2-PROTEIN SPEZIFISCHE A/BETA T-ZELL REZEPTOREN**
METHOD FOR THE RATIONAL MUTAGENESIS OF A/BETA T CELL RECEPTORS AND CORRESPONDINGLY MUTATED MDM2 PROTEIN-SPECIFIC A/BETA T-CELL RECEPTORS
PROCEDE DE MUTAGENESE RATIONNELLE DE RECEPTEURS DE LYMPHOCYTES T A/BETA ET RECEPTEURS DE LYMPHOCYTES T A/BETA SPECIFIQUES DE LA PROTEINE MDM2 AYANT SUBI LADITE MUTAGENESE RATIONNELLE

(30) Priorität: 24.09.2002 DE 10244457
(43) Veröffentlichungstag der Anmeldung: 22.06.2005
(73) Patentinhaber: Johannes Gutenberg-Universität Mainz, 55122 Mainz (DE)
(72) Erfinder: VOSS, Ralf-Holger, 55122 Mainz (DE); MATTHIAS, Theobald, 55122 Mainz (DE)
(74) Vertreter: Krauss, Jan
(86) Internationale Anmeldenummer: PCT/EP2003/010633
(87) Internationale Veröffentlichungsnummer: WO 2004/029085

(56) Entgegenhaltungen:
- WO-A-96/27011
- WO-A-99/16867
- WO-A-02/070552
- DE-A- 10 109 855
- US-A1- 2002 064 521
- BÄCKSTRÖM B T ET AL: "A motif within the T cell receptor alpha chain constant region connecting peptide domain controls antigen responsiveness." IMMUNITY. UNITED STATES NOV 1996, Bd. 5, Nr. 5, November 1996 (1996-11), Seiten 437-447, XP002272013 ISSN: 1074-7613
- STANISLAWSKI T ET AL: "CIRCUMVENTING TOLERANCE TO A HUMAN MDM2-DERIVED TUMOR ANTIGEN BY TCR GENE TRANSFER" NATURE IMMUNOLOGY, NATURE PUBLISHING GROUP,, GB, Bd. 2, Nr. 10, Oktober 2001 (2001-10), Seiten 962-970, XP001086673 ISSN: 1529-2908
- LI Z G ET AL: "Structural mutations in the constant region of the T-cell antigen receptor (TCR)beta chain and their effect on TCR alpha and beta chain interaction." IMMUNOLOGY. ENGLAND AUG 1996, Bd. 88, Nr. 4, August 1996 (1996-08), Seiten 524-530, XP008028134 ISSN: 0019-2805
- CHANG H-C ET AL: "A GENERAL METHOD FOR FACILITATING HETERODIMERIC PAIRING BETWEEN TWO PROTEINS: APPLICATION TO EXPRESSION OF ALPHA AND BETA T-CELL RECEPTOR EXTRACELLULAR SEGMENTS" PROCEEDINGS OF THE NATIONAL ACADEMY OF SCIENCES OF USA, NATIONAL ACADEMY OF SCIENCE. WASHINGTON, US, Bd. 91, Nr. 24, 1. November 1994 (1994-11-01), Seiten 11408-11412, XP002008362 ISSN: 0027-8424

### SEQUENCE LISTING

<110> Johannes Gutenberg Universität Mainz
<120> Verfahren zur rationalen Mutagenese von alpha/beta T-Zell Rezeptoren und entsprechend mutierte hdm2-Protein spezifische alpha/beta T-Zell Rezeptoren
<130> U30031
<160> 6
<170> PatentIn version 3.1
<210> 1
   <211> 200
   <212> PRT
   <213> mus musculus
<400> 1
   Gln Ser Val Thr Gln Pro Asp Ala Arg Val Thr Val Ser Glu Gly Ala
   1               5                   10                  15
   Ser Leu Gln Leu Arg Cys Lys Tyr Ser Tyr Ser Ala Thr Pro Tyr Leu
               20                  25                  30
   Phe Trp Tyr Val Gln Tyr Pro Gln Gly Leu Gln Leu Leu Leu Lys Tyr
           35                  40                  45
   Tyr Ser Gly Asp Pro Val Val Gln Gly Val Asn Gly Phe Glu Ala Glu
       50                  55                  60
   Phe Ser Lys Ser Asn Ser Ser Phe His Leu Arg Lys Ala Ser Val His
   65                  70                  75                  80
   Trp Ser Asp Ser Ala Val Tyr Phe Cys Ala Val Ser Gly Phe Ala Ser
                   85                  90                  95
   Ala Leu Thr Phe Gly Ser Gly Thr Lys Val Ile Val Leu Pro Tyr Ile
               100                 105                 110
   Gln Asn Pro Glu Pro Ala Val Tyr Ala Leu Lys Asp Pro Arg Ser Gln
           115                 120                 125
   Asp Ser Thr Leu Cys Leu Phe Thr Asp Phe Asp Ser Gln Ile Asn Val
       130                 135                 140
   Pro Lys Thr Met Glu Ser Gly Thr Phe Ile Thr Asp Ala Thr Val Leu
   145 150 155 160
   Asp Met Lys Ala Met Asp Ser Lys Ser Asn Gly Ala Ile Ala Trp Ser
   165 170 175
   Asn Gln Thr Ser Phe Thr Cys Gln Asp Ile Phe Lys Glu Thr Asn Ala
   180 185 190
   Thr Tyr Pro Ser Ser Asp Val Pro
   195 200
<210> 2
   <211> 236
   <212> PRT
   <213> Mus musculus
<400> 2
   Met Lys Arg Leu Leu Cys Ser Leu Leu Gly Leu Leu Cys Thr Gln Val
   1               5                   10                  15
   Cys Trp Val Lys Gly Gln Gln Val Gln Gln Ser Pro Ala Ser Leu Val
               20                  25                  30
   Leu Gln Glu Gly Glu Asn Ala Glu Leu Gln Cys Asn Phe Ser Ser Thr
           35                  40                  45
   Ala Thr Arg Leu Gln Trp Phe Tyr Gln Arg Pro Gly Gly Ser Leu Val
       50                  55                  60
   Ser Leu Leu Tyr Asn Pro Ser Gly Thr Lys His Thr Gly Arg Leu Thr
   65                  70                  75                  80
   Ser Thr Thr Val Thr Lys Glu Arg Arg Ser Ser Leu His Ile Ser Ser
                   85                  90                  95
   Ser Gln Thr Thr Asp Ser Gly Thr Tyr Phe Cys Ala Thr Ser Ser Val
               100                 105                 110
   Asn Thr Gly Asn Tyr Lys Tyr Val Phe Gly Ala Gly Thr Arg Leu Lys
           115                 120                 125
   Val Ile Ala His Ile Gln Asn Pro Glu Pro Ala Val Tyr Gln Leu Lys
       130                 135                 140
   Asp Pro Arg Ser Gln Asp Ser Thr Leu Cys Leu Phe Thr Asp Phe Asp
   145                 150                 155                 160
   Ser Gln Ile Asn Val Pro Lys Thr Met Glu Ser Gly Thr Phe Ile Thr
                   165                 170                 175
   Asp Lys Thr Val Leu Asp Met Lys Ala Met Asp Ser Lys Ser Asn Gly
               180                 185                 190
   Ala Ile Ala Trp Ser Asn Gln Thr Ser Phe Thr Cys Gln Asp Ile Phe
           195                 200                 205
   Lys Glu Thr Asn Ala Thr Tyr Pro Ser Ser Asp Val Pro Cys Asp Ala
       210                 215                 220
   Thr Leu Thr Glu Lys Ser Phe Glu Thr Asp Met Asn
   225                 230                 235
<210> 3
   <211> 196
   <212> PRT
   <213> homo sapiens
<400> 3
   Gln Gln Val Lys Gln Asn Ser Pro Ser Leu Ser Val Gln Glu Gly Arg
   1               5                   10                  15
   Ile Ser Ile Leu Asn Cys Asp Tyr Thr Asn Ser Met Phe Asp Tyr Phe
               20                  25                  30
   Leu Trp Tyr Lys Lys Tyr Pro Ala Glu Gly Pro Thr Phe Leu Ile Ser
           35                  40                  45
   Ile Ser Ser Ile Lys Asp Lys Asn Ala Asp Gly Arg Phe Thr Val Phe
       50                  55                  60
   Leu Asn Lys Ser Ala Lys His Leu Ser Leu His Ile Val Pro Ser Gln
   65                  70                  75                  80
   Pro Gly Asp Ser Ala Val Tyr Phe Cys Ala Ala Met Glu Gly Ala Gln
                   85                  90                  95
   Lys Leu Val Phe Gly Gln Gly Thr Arg Leu Thr Ile Asn Pro Asn Ile
               100                 105                 110
   Gln Asn Pro Asp Pro Ala Val Tyr Gln Leu Arg Asp Ser Lys Ser Ser
           115                 120                 125
   Asp Lys Ser Val Cys Leu Phe Thr Asp Phe Asp Ser Gln Thr Asn Val
       130                 135                 140
   Ser Gln Ser Lys Asp Ser Asp Val Tyr Ile Thr Lys Thr Val Leu Asp
   145                 150                 155                 160
   Met Asp Ser Met Asp Phe Lys Ser Asn Ser Ala Val Ala Trp Ser Asn
                   165                 170                 175
   Lys Ser Asp Phe Ala Cys Ala Asn Ala Phe Asn Asn Ser Ile Ile Pro
               180                 185                 190
   Glu Asp Thr Phe
           195
<210> 4
   <211> 236
   <212> PRT
   <213> mus musculus
<400> 4
   Glu Ala Ala Val Thr Gln Ser Pro Arg Asn Lys Val Ala Val Thr Gly
   1               5                   10                  15
   Gly Lys Val Thr Leu Ser Cys Asn Gln Thr Asn Asn His Asn Asn Met
               20                  25                  30
   Tyr Trp Tyr Arg Gln Asp Thr Gly His Gly Leu Arg Leu Ile His Tyr
           35                  40                  45
   Ser Tyr Gly Ala Gly Ser Thr Glu Lys Gly Asp Ile Pro Asp Gly Tyr
       50                  55                  60
   Lys Ala Ser Arg Pro Ser Gln Glu Asn Phe Ser Leu Ile Leu Glu Leu
   65                  70                  75                  80
   Ala Thr Pro Ser Gln Thr Ser Val Tyr Phe Cys Ala Ser Gly Gly Gly
                   85                  90                  95
   Gly Thr Leu Tyr Phe Gly Ala Gly Thr Arg Leu Ser Val Leu Glu Asp
               100                 105                 110
   Leu Arg Asn Val Thr Pro Pro Lys Val Ser Leu Phe Glu Pro Ser Lys
           115                 120                 125
   Ala Glu Ile Ala Asn Lys Gln Lys Ala Thr Leu Val Cys Leu Ala Arg
       130                 135                 140
   Gly Phe Phe Pro Asp His Val Glu Leu Ser Trp Trp Val Asn Gly Lys
   145                 150                 155                 160
   Glu Val His Ser Gly Val Ser Thr Asp Pro Gln Ala Tyr Lys Glu Ser
                   165                 170                 175
   Asn Tyr Ser Tyr Cys Leu Ser Ser Arg Leu Arg Val Ser Ala Thr Phe
               180                 185                 190
   Trp His Asn Pro Arg Asn His Phe Arg Cys Gln Val Gln Phe His Gly
           195                 200                 205
   Leu Ser Glu Glu Asp Lys Trp Pro Glu Gly Ser Pro Lys Pro Val Thr
       210                 215                 220
   Gln Asn Ile Ser Ala Glu Ala Trp Gly Arg Ala Asp
   225                 230                 235
<210> 5
   <211> 307
   <212> PRT
   <213> mus musculus
<400> 5
   Leu Met Asn Lys Trp Val Phe Cys Trp Val Thr Leu Cys Leu Leu Thr
   1               5                   10                  15
   Val Glu Thr Thr His Gly Asp Gly Gly Ile Ile Thr Gln Thr Pro Lys
               20                  25                  30
   Phe Leu Ile Gly Gln Glu Gly Gln Lys Leu Thr Leu Lys Cys Gln Gln
           35                  40                  45
   Asn Phe Asn His Asp Thr Met Tyr Trp Tyr Arg Gln Asp Ser Gly Lys
       50                  55                  60
   Gly Leu Arg Leu Ile Tyr Tyr Ser Ile Thr Glu Asn Asp Leu Gln Lys
   65 70 75 80
   Gly Asp Leu Ser Glu Gly Tyr Asp Ala Ser Arg Glu Lys Lys Ser Ser
   85 90 95
   Phe Ser Leu Thr Val Thr Ser Ala Gln Lys Asn Glu Met Ala Val Phe
   100 105 110
   Leu Cys Ala Ser Gly Asp Trp Gly Tyr Glu Gln Tyr Phe Gly Pro Gly
   115 120 125
   Thr Arg Leu Thr Val Leu Glu Asp Leu Arg Asn Val Thr Pro Pro Lys
   130 135 140
   Val Ser Leu Phe Glu Pro Ser Lys Ala Glu Ile Ala Asn Lys Gln Lys
   145 150 155 160
   Ala Thr Leu Val Cys Leu Ala Arg Gly Phe Phe Pro Asp His Val Glu
   165 170 175
   Leu Ser Trp Trp Val Asn Gly Lys Glu Val His Ser Gly Val Ser Thr
   180 185 190
   Asp Pro Gln Ala Tyr Lys Glu Ser Asn Tyr Ser Tyr Cys Leu Ser Ser
   195 200 205
   Arg Leu Arg Val Ser Ala Thr Phe Trp His Asn Pro Arg Asn His Phe
   210 215 220
   Arg Cys Gln Val Gln Phe His Gly Leu Ser Glu Glu Asp Lys Trp Pro
   225 230 235 240
   Glu Gly Ser Pro Lys Pro Val Thr Gln Asn Ile Ser Ala Glu Ala Trp
   245 250 255
   Gly Arg Ala Asp Cys Gly Ile Thr Ser Ala Ser Tyr His Gln Gly Val
   260 265 270
   Leu Ser Ala Thr Ile Leu Tyr Glu Ile Leu Leu Gly Lys Ala Thr Leu
   275 280 285
   Tyr Ala Val Leu Val Ser Gly Leu Val Leu Met Ala Met Val Lys Lys
   290 295 300
   Lys Asn Ser
   305
<210> 6
   <211> 244
   <212> PRT
   <213> homo sapiens
<400> 6
   Asn Ala Gly Val Thr Gln Thr Pro Lys Phe Gln Val Leu Lys Thr Gly
   1               5                   10                  15
   Gln Ser Met Thr Leu Gln Cys Ala Gln Asp Met Asn His Glu Tyr Met
               20                  25                  30
   Ser Trp Tyr Arg Gln Asp Pro Gly Met Gly Leu Arg Leu Ile His Tyr
           35                  40                  45
   Ser Val Gly Ala Gly Ile Thr Asp Gln Gly Glu Val Pro Asn Gly Tyr
       50                  55                  60
   Asn Val Ser Arg Ser Thr Thr Glu Asp Phe Pro Leu Arg Leu Leu Ser
   65                  70                  75                  80
   Ala Ala Pro Ser Gln Thr Ser Val Tyr Phe Cys Ala Ser Ser Tyr Pro
                   85                  90                  95
   Gly Gly Gly Phe Tyr Glu Gln Tyr Phe Gly Pro Gly Thr Arg Leu Thr
               100                 105                 110
   Val Thr Glu Asp Leu Lys Asn Val Phe Pro Pro Glu Val Ala Val Phe
           115                 120                 125
   Glu Pro Ser Glu Ala Glu Ile Ser His Thr Gln Lys Ala Thr Leu Val
       130                 135                 140
   Cys Leu Ala Thr Gly Phe Tyr Pro Asp His Val Glu Leu Ser Trp Trp
   145                 150                 155                 160
   Val Asn Gly Lys Glu Val His Ser Gly Val Ser Thr Asp Pro Gln Pro
   165 170 175
   Leu Lys Glu Gln Pro Ala Leu Asn Asp Ser Arg Tyr Ala Leu Ser Ser
   180 185 190
   Arg Leu Arg Val Ser Ala Thr Phe Trp Gln Asp Pro Arg Asn His Phe
   195 200 205
   Arg Cys Gln Val Gln Phe Tyr Gly Leu Ser Glu Asn Asp Glu Trp Thr
   210 215 220
   Gln Asp Arg Ala Lys Pro Val Thr Gln Ile Val Ser Ala Glu Ala Trp
   225 230 235 240
   Gly Arg Ala Asp

## Beschreibung

Die Erfindung betrifft die rationale Mutagenese von Polypeptiden von Onkogen-spezifische T-Zell Antwort vermittelnden α/β T-Zell Rezeptoren, diese kodierende Nukleinsäuren und deren Verwendung bei der Therapie, Diagnose und/oder Prävention von Krebserkrankungen. Die Erfindung betrifft weiterhin einen mittels des Verfahrens der vorliegenden Erfindung rational mutierten MDM2-Protein-spezifische T-Zell Antwort vermittelnden α/β T-Zell Rezeptoren und dessen Verwendungen.

Die Antigenerkennung durch T-Lymphozyten (TZL) ist entscheidend für die Erzeugung und Regulierung einer effektiven Immunantwort. Der charakteristische T-Zelllinien-Marker ist der T-Zell-Antigen-Rezeptor (TZR). Es gibt zwei sequenzverschiedene Typen von TZR: die heterodimeren α/β-TZR und die strukturell verwandten γ/δ-TZR. Die jeweiligen Kettenpaare sind über eine Disulfidbrücke kovalent verknüpft und sind mit einem Set von fünf Polypeptiden, dem CD3-Komplex assoziiert und bilden so zusammen den T-Zell Rezeptor-Komplex (TZR-CD3-Komplex). Der α/β-TZR ist der funktionell bedeutendste, da er in über 95% aller T-Zellen exprimiert wird und die tragende Immunantwort vermittelt.

α/β-T-Zellen können in zwei verschiedene sich überschneidende Populationen unterteilt werden: Eine Untergruppe, die den CD4-Marker trägt und hauptsächlich die Immunantwort unterstützt (T_{H}) und eine Untergruppe, die den CD8-Marker trägt und im wesentlichen zytotoxisch ist (T_{C}). CD8⁺-T-Zellen erkennen Antigene in Assoziation mit MHC-Klasse-I-Molekülen. Solche Antigene können unter anderem tumorspezifische oder tumorassoziierte Peptidantigene sein. Nach Erkennung der Peptidantigene wird die betreffende Zelle abgetötet, indem die T-Zelle die Zielzelle lysiert und/oder Apoptose dieser Zielzellen induziert oder Zytokine (z.B. IL-2, IFN-γ) freisetzt. Hier liegt ein wesentlicher funktioneller Unterschied zu Antikörpern: TZR erkennen ausschließlich Peptidantigene im Kontext der MHC-Präsentation, wohingegen Antikörper von weiteren akzessorischen Molekülen unabhängige sequentielle oder konformationelle Peptidantigene erkennen. TZR sind daher die geeigneten molekularen Werkzeuge, Tumorprotein-abgeleitete Antigene zu erkennen und direkt an eine cytotoxische T-Zell-Antwort zu koppeln. Antikörper hingegen dienen in erster Linie dazu, Oberflächenmarker von Zellen als pathogen zu erkennen und sie zu markieren, um in Folge von anderen Effektorzellen z.B. von Makrophagen im Wege der Phagozytose eliminiert zu werden.

Unter den tumorassoziierten Peptidantigenen (TAA), die im Kontext von MHC-Klasse-I-Molekülen auf der Oberfläche von Tumorzellen präsentiert werden, sind die sogenannten "universellen" TAA von besonderem Interesse. Diese TAA leiten sich überwiegend von zellulären Proteinen ab, die in normalen Zellen schwach exprimiert und in Tumorzellen überexprimiert werden. Zu diesen Proteinen gehört unter anderem das humane Homolog des "Mouse-Double-Minute-2" Proto-Onkogens (mdm2), das sogenannte "humane mdm2" oder abgekürzt "MDM2" Proto-Onkoprotein (Roth et al. 1998), das nicht nur in einer Reihe solider Tumore, sondern auch bei den hämatologischen Neoplasien (malignen hämatologischen Systemerkrankungen) AML, ALL und CLL überexprimiert wird (Zhou et al, 2000).

Oligopeptide des MDM2-Proteins können im Kontext mit MHC-Klasse-I-Molekülen auf der Zelloberfläche präsentiert werden und repräsentieren attraktive Zielstrukturen für CD8-positive T-Zellen. Das Ausmaß der T-Zell Antwort verläuft hierbei in einem definierten kinetischen Fenster (Kersh et al., 1998). Der Komplex aus Peptid-MHC und TZR-CD3 multimerisiert, um eine effiziente Signaltransduktion zu bewirken, wobei die genaue Stöchiometrie und das Ausmaß der Oligomerisierung noch umstritten ist.

Die vorliegende Erfindung behandelt ein biochemisches Problem auf dem Feld der angewandten Immunologie & Onkologie: Focus der Erfindung ist die Entwicklung hocheffektiver T-Zellen, die über ihre cytotoxische Effektor-Funktion in der Lage sind, humane (Hu) Tumorzellen spezifisch zu erkennen und zu lysieren. Dazu werden CTL-Klone, die spezifisch TAA erkennen, im transgenen murinen (Mu) oder Maus-Modell isoliert (Stanislawski & Voss et al., 2001). Verantwortlich für die Onkoprotein abgeleitete PeptidErkennung ist der membranständige TZR auf Seite der T-Zelle, der den Komplex aus membranständigem MHC-Molekül und präsentiertem Peptid, das aus der proteosomalen Prozessierung von Onkoproteinen hervorgegangen ist, auf Seite der Antigen präsentierenden Zelle (APC), oder auch Tumorzelle, erkennt und ein aktivierendes Signal an die Signaltransduktionskaskade der cytotoxischen T-Zelle (CTL) vermittelt.

Die prospektive klinische Anwendung sieht dann vor, periphere T-Zellen des Blutes eines Tumorpatienten zu isolieren, diesen die Gene der TAA-spezifischen TZR durch den adoptiven, gentechnischen Transfer hinzuzufügen und nach massiver Expansion zu reinfundieren (Rosenberg, 1999; Schumacher, 2002).

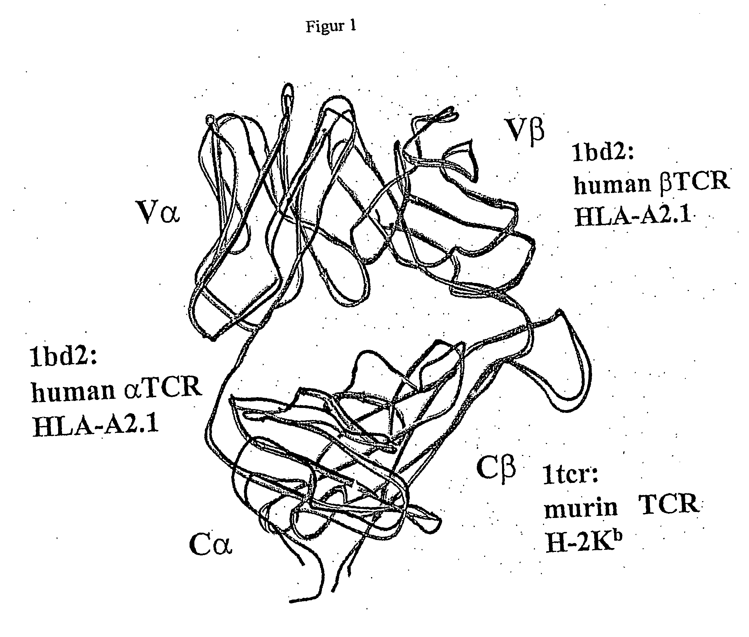

Der T-Zell-Rezeptor ist ein heterodimeres α/β-Molekül, dessen Ketten jeweils die Transmembrane einmalig durchspannen. Jede Kette besteht aus zwei globulären Domänen, die eine Immunglobulin-ähnliche Faltung vornehmen: die aminoterminale Domäne wird als die variable Domäne (Vα bzw Vβ) bezeichnet, da diese aus genetischem Rearrangement hervorgegangen ist und für die individuelle Peptiderkennung zuständig ist. Die sich anschließende Domäne wird die invariante oder auch konstante Domäne (Cα bzw Cβ) genannt, da diese streng konserviert ist und im wesentlichen eine Abstandshalterfunktion der variablen Domäne zur Zellmembran innehat sowie regulatorische Proteine an ihr binden. Zuletzt kommt der Abschnitt einer transmembranen Region und eines kurzen carboxyterminalen cytoplasmatischen Endes, an denen der signaltransduzierende CD3-Komplex binden kann. Dieser Aufbau gilt gleichermaßen für humane als auch murine TZR: das Protein-Rückgrat der Spezies-fremden TZR ist mit einem marginalen Unterschied von nur 1,04 Angström superpositionierbar. Insbesondere in der konstanten Domäne sind zahlreiche Aminosäuren homolog bzw. identisch (siehe Figur 1), wie exemplarisch gezeigt für einen murinen (1tcr) zu einem humanen, HLA-A2.1-restringierten TCR (1bd2). Der T-Zell-Rezeptor ist zwar ein heterodimeres Molekül aus zwei Polypeptid-Ketten, besitzt aber im Gegensatz zum Antikörper eine monovalente Bindungsstelle. Antikörper sind prinzipiell Homodimere von heterodimeren Untereinheiten, aus der sich eine bivalente Antigenerkennung ein und desselben Antigens ergibt (d.h. monospezifisch): zwei identische Arme, jeweils bestehend aus einer heterodimeren Aneinanderlagerung einer schweren und einer leichten Kette mit einer sich formierenden Antigen-Bindungsstelle, sind über eine Disulfidbrücke kovalent miteinander verknüpft. Daraus ergeben sich Kontaktflächen zwischen den Armen eines Antikörpers, die es beim TZR nicht gibt: hieraus entstanden Fragestellungen (Atwell et al., 1997; WO96/27011), die bivalente Antigenerkennung dahingehend zu verändern, jede der beiden Antigen-Bindungsstellen ein unterschiedliches Antigen erkennen zu lassen, also eine Bispezifität im gesamten Antikörper-Molekül einzuführen. Dies dient letzten Endes beispielhaft dazu, daß ein Antikörper sowohl ein gewebsspezifisches Antigen als auch ein pathogenes Antigen erkennt. Dieses Ziel ist beim TZR nicht realisierbar, da dieser strukturell nur einem der beiden Arme eines Antikörpers entspricht, also monovalent und damit zwangsläufig monospezifisch ist.

Eine andersgeartete Fragestellung ergibt sich für die therapeutische Anwendung von TZR: dieses besteht darin, daß die polyklonale T-Zellpopulation des Patienten eigene, sogenannte endogene, T-Zell-Rezeptoren unbekannter Spezifität trägt. Ein funktioneller TZR formiert sich aus der Paarung der beiden Ketten im Wege des Endoplasmatischen Retikulum (ER)-und Golgi-prozessonalen Vorganges zu einem an die Zell-Oberfläche geleiteten TZR/CD3-Komplexes. Da die heterodimeren Ketten zunächst vereinzelt exprimiert werden und zudem hinreichende Homologie zwischen den humanen und murinen Ketten bestehen, können die exogen hinzugefügten TZR mit den endogenen Ketten paaren und unbekannte, im ungünstigen Fall ungewünschte Monospezifitäten (Autoimmun-Reaktionen) hervorrufen. Im einfachsten Fall ergeben sich aus den vier vorhandenen Ketten (zwei endogene und zwei exogene Ketten) einer mit den Genen für einen TZR-transduzierten T-Zelle vier denkbare Kombinationen, von denen zwei ungewünschte Hu αTZR/Mu βTZR und Mu αTZR/Hu βTZR - Hybride sind. Die Situation verkompliziert sich zusätzlich dann, wenn mehrere, für verschiedene TAA spezifische TZR-Gene transduziert werden bzw. dadurch, daß zuweilen in einer T-Zelle zwei funktionelle TZR aufgrund eines insuffizienten allelischen Ausschlusses der einen von zwei genomischen α-Ketten exprimiert werden. Zudem werden im Rahmen einer klinischen Applikation voraussichtlich nicht-klonale T-Zell-Populationen transduziert, so daß hieraus eine Vielzahl denkbarer hybrider TZR auf Populationsebene vorliegen.

Eine solche hybride Form ist bisher nicht bewiesen worden, kann aber aufgrund der bisher vorliegenden strukturellen Daten wie TZR-Protein-Kristallstrukturen (Garcia et al., 1998; Ding et al., 1998) nicht ausgeschlossen werden. Es konnte aber im Labor der Erfinder gezeigt werden, daß eine fremde, einzelne Maus-TCRβ-Kette, die in humane T-Zellen eingeschleust wird, nur dann an der Oberfläche exprimiert wird, wenn diese in der Lage ist mit den endogenen humanen TCRα-Ketten zu paaren. Da dies nachweisbar war und die exogene Expression im Fall partiell humanisierter TCRβ-Ketten verstärkbar, bzw. durch das Einführen von Punktmutationen, die das Paaren behindern (siehe unten beschriebener Ansatz "kompensierte Einführung eines exponierten Ladungsträgers"), abschwächbar war, ist dies ein starkes Indiz für das Vorhandensein hybrider TZR. Zu einer erhöhten Sicherheit bei der Anwendung von TZR gilt es daher, solche Hybridformen so weit wie möglich auszuschließen.

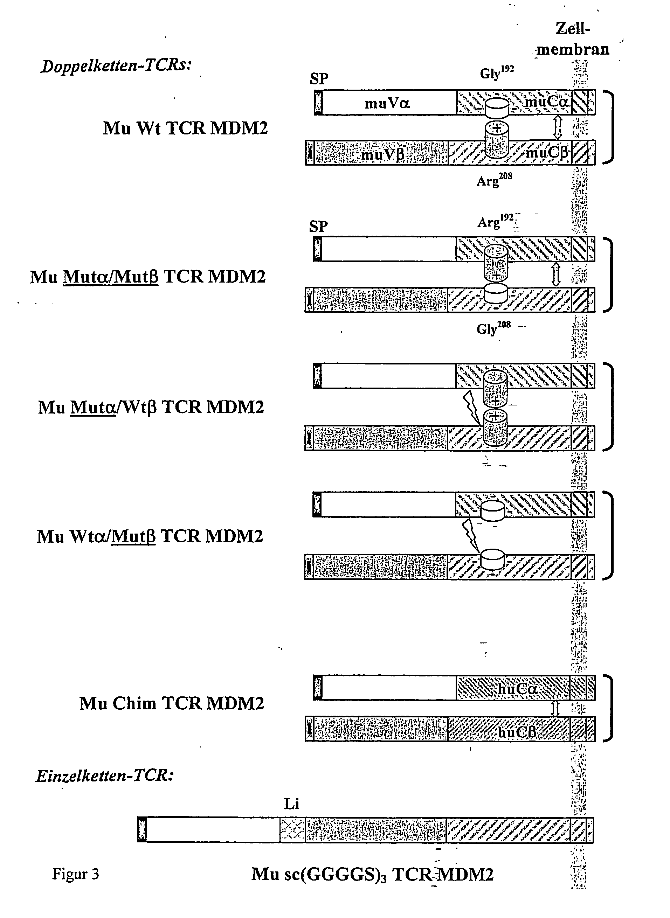

Ein Lösungsweg zur Umgehung der ungewünschten Kettenpaarung ist das Design von Einzelketten-T-Zell-Rezeptoren. Dieser Ansatz wurde ursprünglich für Antikörper entwickelt (Eshhar et al., 1993; 2001), konnte aber aufgrund der strukturellen Homologien zwischen Antikörpern und T-Zell-Rezeptoren auf letztere übertragen werden (Chung et al., 1994; Weijtens et al., 1998; Willemsen et al., 2000). Hierbei werden die variablen Domänen über ein kurzes Peptid, einen "Linker", miteinander kovalent unter Wegfall der einen von beiden konstanten Domänen verknüpft. Solche Konstrukte sind gentechnisch beliebig konzipierbar und gewährleisten eine biochemisch gekoppelte 1:1-Stöchiometrie der heterodimeren, variablen Domänen (siehe dazu auch Figur 3).

Alternativ werden an die jeweiligen Ketten des heterodimeren Moleküls kurze Peptidsequenzen als Affinitäts-anhängsel gentechnisch angefügt, die für eine spezifische Kettenpaarung Sorge tragen: hierzu wurden T-Zell-Rezeptoren 30 Aminosäure lange, carboxyterminale "tags", ein sogenannter "leucine zipper", als Dimerisierungsmotiv angefügt (Chang et al., 1994). Diese letztgenannten Verfahren haben den Nachteil, aufgrund ihres rekombinanten Charakters potentielle Fremdantigene darzustellen, die zu Abstoßungsreaktionen im Empfängerorganismus führen.

Zudem kann die modifizierte Primär- und Tertiärstruktur, die deutlich von der Wildtypstruktur humaner als auch muriner TZR abweicht, die Stabilität und Funktionalität dieser chimären Konstrukte beeinträchtigen.

WO 02/070552 beschreibt die Humanisierung eines TZR durch Austausch der murinen durch humane konstante Domänen. Es werden dabei Mutationen eingeführt, die durch Austausch von ganzen Blöcken anderer Ketten eingeführt werden. Es wird kein Schlüssel-Schloss-Prinzip beschrieben, die Paarung der Ketten wird auch nicht selektiv gefördert, sondern es wird versucht, immunologische Komplikationen mit dem murinen Antikörper zu vermeiden.

Der vorliegenden Erfindung liegt daher die Aufgabe zugrunde, ein Verfahren zur Verfügung zu stellen, das die Herstellung rekombinanter TZR ermöglicht, so daß bevorzugt die von außen zugeführten TZR-Ketten paaren und nicht gemischte Pärchen mit den endogenen Ketten der T-Zellen ausbilden, ohne dabei deren Funktionalität und Stabilität zu beeinträchtigen.

Erfingdungsgemäß wird diese Aufhabe durch ein Verfahren zur Herstellung eines rekombinanten heterodimeren spezifischen Wildtyp- oder chimären T-Zell Rezeptors (TZR), so dass bevorzugt die von außen zugeführten TZR-Ketten paaren und nicht gemischte Pärchen mit den endogenen Ketten der T-Zellen ausbilden, ohne dabei deren Funktionalität und Stabilität zu beeinträchtigen, wobei der TZR eine erste Kette und eine zweite Kette enthält, die miteinander an mindestens einer Oberfläche ihrer Aminosäuren wechselwirken wobei die mindestens eine Aminosäuren-Oberfläche einer rationalen Mutagenese unterzogen wird, so dass die mindestens eine Aminosäuren-Oberfläche der ersten Kette oder die Aminosäuren-Oberfläche der zweiten Kette eine räumlich hervorstehende chemische Gruppe umfaßt, die mit einer räumlich zurückgesetzte chemischen Gruppe auf der mindestens einen Oberfläche der im TZR entsprechend gepaarten ersten Kette oder zweiten Kette wechselwirkt, gelöst.

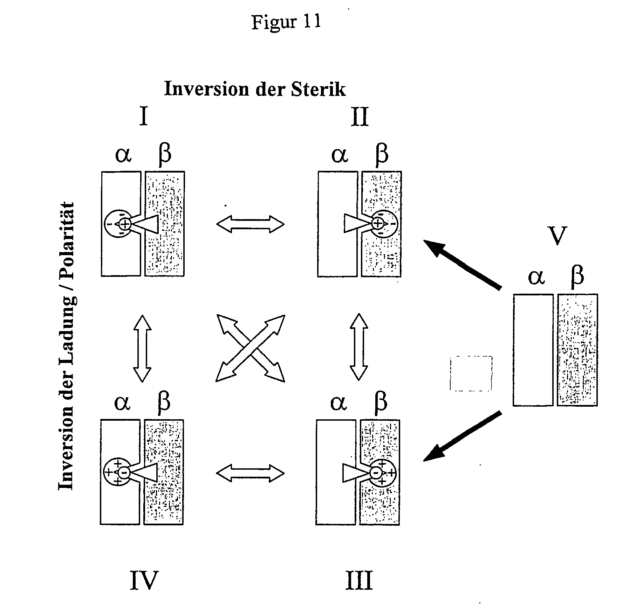

Bevorzugt ist die von der mindestens einen Oberfläche der ersten Kette oder der Oberfläche der zweiten Kette umfaßte räumlich hervorstehende Gruppe eine geladene und/oder polare Gruppe, und weiterhin bevorzugt ist die räumlich zurückgesetzte Gruppe auf der mindestens einen Oberfläche der entsprechenden ersten Kette oder zweiten Kette eine entgegengesetzt geladene und/oder polare Gruppe. Hierbei kann es genügen, eine exponierte Ladung einseitig zu inserieren, wenn die gegenüberliegende Kontaktfläche diese Veränderung kooperativ kompensieren kann oder aber es erfordern, eine entgegengesetzte zurückversetzte Ladung komplementär einzuführen. Im folgenden soll dieser neuartige Ansatz mit der "kompensierten Einführung eines exponierten Ladungsträgers" bezeichnet und beschrieben werden (Figur 11). Jedes "+" - als auch "-" - Symbol beschreibt eine echte Ladung oder eine polare Ladung. Eine echte Ladung (z.B. die geladene Guanidinium-Gruppe von Arginin) kann erst durch mehrere polare Gruppen (z.B. Carbonyle der Peptidgruppen) mehrerer Oberflächen (Aminosäuren) hinreichend kompensiert werden. Dies wird durch die nicht-stöchiometrische Darstellung der Ladungssymbole angedeutet: in vielen Fällen zeigt eine sterisch exponierte echte Ladung der einen Kette (def. als räumlich hervorstehende Gruppe) in eine Höhle der anderen Kette (def. als räumlich zurückversetzte Gruppe), die mit mehreren polaren oder auch geladenen Gruppen ausgekleidet ist. Selbst echte Ladungen kompensieren sich nicht direkt 1:1, da dies auch von der betreffenden (dielektrischen) Zugänglichkeit und Entfernung benachbarter entgegengesetzter Ladungen abhängt. Sowohl in Hinblick auf Sterik als auch Ladung müssen die betreffenden Gruppen nicht direkt 1:1 umgesetzt invertiert werden, sondern dies bedarf einer individuellen strukturellen Analyse, ggfls. z.B unter Wegfall einer polaren Gruppe innerhalb der die Ladungsträger beinhaltenden Höhle, um einen möglichst kompensatorischen Effekt zu erzielen. Nicht nur der Zustand V, in dem eine betreffende invers bezogene Ladungsträger-Oberfläche auf den jeweiligen Ketten fehlt, entspricht hierbei dem Wildtyp, sondern jeder der Zustande 1-IV kann ebenso dem vorgefundenen Ausgangszustand der Polypeptidketten entsprechen.

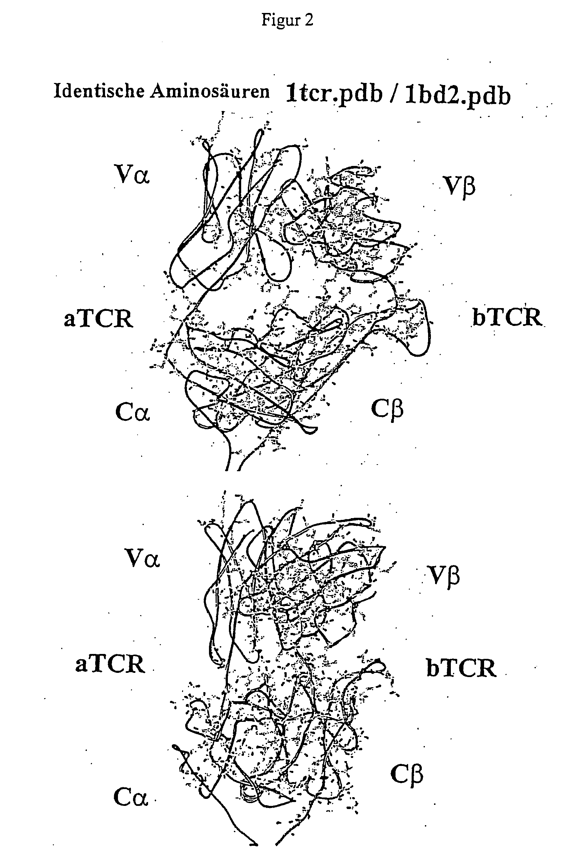

Hierbei besteht die Möglichkeit, ausschließlich die gegebenen sterischen Verhältnisse zu invertieren (I/II bzw. III/IV in Figur 11), die Ladungen zu invertieren (I/IV bzw II/III in Figur 11) oder beides (II/IV bzw. I/III in Figur 11). Der besondere Fall der Inversion der geladenen und/oder polaren Sterik (I/II der Figur 11) dient als an T-Zell-Rezeptoren experimentell belegtes Beispiel (Figur 2).

Das erfindungsgemäße Verfahren umfaßt die Schritte von (a) zu Verfügung stellen der DNA-Moleküle, die die kodierenden Bereiche für die mindestens eine zu mutierende Aminosäuren-Oberfläche der ersten Kette oder zweiten Kette umfassen, in einem gemeinsamen oder getrennten Mutagenese-Vektorsystem(en), (b) Mutagenese der DNA-Moleküle auf an sich bekannte Weise, wobei die für die mindestens eine Aminosäuren-Oberfläche kodierende Nukleinsäuresequenz im Vergleich zu der Ausgangssequenz so verändert wird, dass in die mindestens eine Aminosäuren-Oberfläche der ersten Kette oder die mindestens eine Aminosäuren-Oberfläche der zweiten Kette gegenüber der ursprünglichen Aminosäuren-Oberfläche eine räumlich hervorstehende chemische Gruppe eingeführt wird, und in die entsprechend in Wechselwirkung stehende mindestens eine Aminosäure-Oberfläche der zweiten Kette oder der ersten Kette gegenüber der ursprünglichen Aminosäuren-Oberfläche(n) eine räumlich zurückgesetzte chemische Gruppe eingeführt wird, wodurch einzelne Mutanten-Fragmente hergestellt werden, wobei die nach der Mutagenese der DNA-Moleküle eingeführte Aminosäure, die gegenüber der ursprünglichen Aminosäuren-Oberfläche eine räumlich zurückgesetzte Gruppe einführt, ausgewählt ist aus Glyzin, Serin, Threonin, Valin und Alanin und wobei die nach der Mutagenese der DNA-Moleküle eingeführte Aminosäure, die gegenüber der ursprünglichen Aminosäuren-Oberfläche eine räumlich hervorstehende Gruppe einführt, ausgewählt ist aus Glutamin, Glutaminsäure, alpha-Methylvalin Histidin, Hydroxylysin, Tryptophan, Lysin, Arginin, Phenylalanin und Tyrosin, und c) Translation von mindestens zwei der einzelnen Mutanten-Fragmente aus Schritt b) in vitro oder in isolierten Zellen, so dass die Paarung des heterodimeren, mindestens an einer Aminosäure-Oberfläche mutierten spezifischen ersten-Kette/zweite-Kette TZR selektiv gefördert wird, und d) Präsentation des heterodimeren erste-Kette/zweite-Kette TZR durch eine T-Zelle.

Unter einer "Oberfläche" im Sinne der vorliegenden Erfindung wird der Bereich einer Kette eines TZR verstanden, der mit einem bestimmten Bereich der zweiten Kette des TZR wechselwirkt. Diese Wechselwirkung allein oder in Zusammenhang mit anderen führt zu der Ausbildung von Paaren von Ketten, die aktive TZR bilden. Die Summe der Wechselwirkungen beruht auf elektrostatischen, Dipol-Dipol-, Van der Waals-Kontakten und hydrophoben Wechselwirkungen die durch die Aminosäuresequenzen als auch die strukturellen Anordnungen der Polypeptidketten untereinander und zueinander bestimmt werden. Aufgrund einer punktuellen Veränderung sollte die Sekundär-, Tertiär- und Quartärstruktur des Heterodimers unbeeinflußt bleiben. Ohne auf einen bestimmten Wirkmechanismus festgelegt sein zu wollen, gehen die Erfinder davon aus, daß grundsätzlich durch das Verfahren der vorliegenden Erfindung gezielt auf die Kombination aus Ladung und Sterik von Seitenkette(n) eingewirkt wird, ohne das Protein-Rückgrat zu verändern. Die Modifikation von sterischen Gegebenheiten zwischen interagierenden Oberflächen ist in vielen Fällen nicht ausreichend, da selbst sterisch große Seitenketten (Valin, Phenylalanin) derart die lokale Struktur unter Verdrängen benachbarter Seitenketten, ob nun die eigene Kette (Oberfläche) oder die komplementäre Kette (Oberfläche) betreffend, verändern, daß diese ohne Effekt auf die Ketten- (Oberflächen-)-Paarung räumlich innerhalb der Kontaktfläche untergebracht werden. Einen wirkungsvollen Beitrag leistet die Abstoßung durch Ladungsgegensätze, da punktförmige Ladungen gemäß dem Coulomb-Gesetz sphärisch mit 1/Dr² im Raum wirken. Mit zunehmendem Abstand nimmt die einwirkende Kraft exponentiell ab und sorgt somit für eine lokal restringierte Wirkung der Punktladung ohne weitreichende Auswirkung auf die Tertiär- und Quartärstruktur. Die Dielektrizitätskonstante D ist in apolaren Milieus, wie es im Innern von Proteinen oder auch an Kontaktflächen von Untereinheiten der Fall ist, niedriger und die Kraft die auf entgegengesetzte Ladungen wirkt hiermit eine größere. Um den potentiell durch sterische Abstoßung induzierten (oder auch ausgebliebenen) Effekt auf die Oberflächen-Interaktion zu verstärken (bzw. überhaupt erst zu initiieren), werden Aminosäurereste mit sterisch exponierten Seitenketten mit Ladungen (z.B. Arginin, Lysin, Glutamat), pH-induzierbaren Ladungen (z.B. Histidin) oder polaren Gruppen gewählt (z.B. Glutamin). Dies geschieht bevorzugt durch Veränderungen auf der Primärstruktur-Ebene der Kette, nämlich durch Aminosäureaustausche in derselben. Die punktuelle Wirkung sowohl der Sterik als auch der Ladung zweier interagierender Oberflächen (z.B. zweier interagierender Aminosäuren entgegengesetzter Ladung und reziproker Sterik) gewährleistet die Integrität der Raumstrukturen der betreffenden Ketten selbst, induziert aber durch die räumlich begrenzte Störung im Fall der Interaktion zweier exponierter bzw. zurückversetzter gleichgerichteter geladener Oberflächen, wie es für die Interaktion einer unveränderten mit einer mutierten Kette einträfe, eine Schwächung der Paarung. Die komplementären Oberflächen hingegen neutralisieren sich gegenseitig hinsichtlich Sterik und Ladung.

Eine Alternative des erfindungsgemäßen Verfahrens betrifft ein Verfahren, in dem der oben genannte Schritt c) durch folgende Schritte ersetzt wird: (c') gegebenenfalls Umklonierung der Mutanten-Fragmente in geeignete Transfektions-Vektorsysteme oder Transduktions-Virus-abgeleitete Systeme, (c") Transfektion oder Cotransfektion oder Transduktion mindestens zwei der Mutanten-Fragmente in eine Mutanten TZR-defiziente T-Zelle, und (c"') Expression des heterodimeren erste-Kette/zweite-Kette TZR in einer rekombinanten T-Zelle. Diese Variante betrifft den Transfer des genetischen Konstrukts und dessen anschließende Expression direkt in einer rekombinanten T-Zelle. Während diese Alternative bevorzugt ist, kann in einer weiteren Alternative des erfindungsgemäßen Verfahrens Schritt c) oben durch folgende Schritte ersetzt werden: c') In vitro-Translation oder *in vivo*-Translation von mindestens zwei der einzelnen Mutanten-Fragmente aus Schritt b) und gegebenenfalls anschließende Isolierung und/oder Reinigung der translatierten Mutanten-Fragmente, so daß die Paarung des heterodimeren, mindestens an einer Oberfläche mutierten spezifischen ersten-Kette/zweite-Kette TZR selektiv gefördert wird, und c") Einbringung der mutierten spezifischen ersten-Kette/zweite-Kette TZR in eine T-Zelle.

Dabei erfolgt somit eine Expression des mutierten TZR außerhalb der zuletzt vorgesehenen präsentierenden T-Zelle und eine anschließende Einbringung des TZR in dieselbe. Bei der *in vitro* Translation kann die Translation in zellfreien Systemen erfolgen, die käuflich erhältlich sind. Die "Translation" umfaßt aber auch die rein synthetische Herstellung der Peptidketten, die weiter unten im Rahmen der Peptide genauer erläutert wird. Im Falle der *in vivo* Translation kann diese in einer geeigneten Wirtszelle erfolgen, die vorher mit einem Expressionskonstrukt der Kette transformiert wurde und diese anschließend herstellt. Geeignete Vektoren und Verfahren zur Expression sind dem Fachmann im Stand der Technik ausreichend bekannt. Nach der Expression kann es erforderlich sein, die Expressionsprodukte entweder aus den Zellen zu reinigen oder aus dem Medium zu extrahieren, in das sie gegebenenfalls durch die Wirtszelle exkretiert wurden. Geeignete Wirtszellen sind ebenfalls bekannt und können Hefe, CHO-Zellen, Insektenzellen, Bakterien oder andere sein.

Die Einbringung in die T-Zielzellen kann auf jede bekannte Weise erfolgen, die eine anschließende Präsentation des TZR durch die T-Zelle ermöglicht. Wege sind zum Beispiel über die Induktion von Phagocytose durch die Zellen oder ein Verfahren, wobei die Einbringung durch Lipid-vermittelten Transfer, wie zum Beispiel über Micellen oder Liposomen-Transfer erfolgt. Eine Übersicht über die Verwendung von Liposomen gibt u.a. der Artikel Banerjee R. Liposomes: applications in medicine. J Biomater Appl 2001 Jul;16(1):3-21. Der Transfer über Micellen ist dem Fachmann im Stand der Technik aus zahlreichen Publikationen bekannt.

Bevorzugterweise wird erfindungsgemäß als heterodimerer spezifischer Wildtyp- oder chimären T-Zell Rezeptor (TZR) ein alpha/beta TZR, gamma/delta TZR, ein humanisierter oder partiell humanisierter TZR, ein mit zusätzlichen (funktionellen) Domänen versehener TZR, ein mit alternativen Domänen versehener TZR, wie z.B ein mit einer anderen Transmembran-Domäne als Membrananker versehener TZR modifiziert.

Backstrom et al. (Backstrom BT, Hausmann BT, Palmer E. Signaling efficiency of the T cell receptor controlled by a single amino acid in the beta chain constant region. J Exp Med. 1997 Dec 1;186(11):1933-8) beschreiben eine Gln₁₃₆Phe-Mutation im beta-TZR. Diese liegt im "beta chain connecting peptide domain" nahe an der Transmembrane. Der beschriebene Austausch liegt weitab von den in der vorliegendnen Erfindung als für die rationale Mutagenese ermittelten brauchbaren Punktmutanten. Zudem wird eine Wirkung auf die Paarung der Ketten nicht analysiert, sondern ausschließlich eine Funktionalität der TZR.

Backstrom et al. (Backstrom BT, Milia E, Peter A, Jaureguiberry B, Baldari CT, Palmer E. A motif within the T cell receptor alpha chain constant region connecting peptide domain controls antigen responsiveness. Immunity. 1996 Nov;5(5):437-47.) beschreiben chimäre TZR, deren Fusionspunkte jenseits des terminalen Intra-Ketten - Cysteins liegen und insbesondere ein Motiv der "alpha chain connecting peptide domain" FETDxNLN betraf. Beide Mutationenregionen liegen dicht an der Transmembran-Domäne, also weitab von den von hier als wesentlich beschriebenen Punktmutanten. Reziproke Aminiosäureaustausche (knob-hole) werden nicht durchgeführt.

Li et al. (Li ZG, Wu WP, Manolios N. Structural mutations in the constant region of the T-cell antigen receptor (TZR) beta chain and their effect on TZR alpha and beta chain interaction. Immunology. 1996 Aug;88(4):524-30) und WO 97/47644 und WO 96/22306 beschreiben die Paarungsanalyse von TZR. Die Paarung wird durch Immunpräzipitation und 2D-Geleelektrophorese mit einem vorangegangenen metabolischem Markieren der Ketten analysiert. Die Autoren kreierten chimäre betaTZR zu einem nicht immunologisch relevanten Molekül und untersuchten, ob diese verschieden lang dem Wildtyp der beta-Kette noch entsprechenden Chimäre mit der alpha-Kette paarten. Die Chimären umfassen verschiedene Bereiche insbesondere der konstanten Domäne, bezeichnen aber nicht die im Rahmen der vorliegenden Erfindung beschriebenen Punktmutanten. Die Pairing-Eigenschaften werden weder unter dem Einfluß der Mutagenese auf beiden Ketten noch im Rahmen einer sterischen Inversion (knob-hole) untersucht.

Die Autoren ziehen jedoch zwei für die Erfindung sehr wichtige Schlüsse: Zum einen ist die konstante Domäne maßgeblich für das Pairing verantwortlich, wobei dabei besonders der Bereich Ser₁₈₈-Leu₂₁₃ des betaTZR interessant sei, der besonders viele basische Aminosäuren (Arginin₂₀₈ bzw. Arginin₁₉₅ des 1tcr) enthält und wahrscheinlich wesentlich für das Pairing ist. Darauf wird in dieser Publikation jedoch nicht weiter eingegangen.

Die Fragestellung der spezifischen Manipulierung der Molekül-Interaktion wurde bereits versucht, über sterisch komplementäre Gruppen zu lösen (Belshaw et al., Angew. Chem. Int. Ed. Engl. 34 (1995), 2129-2132) und anhand von Antikörpern exemplifiziert (WO 96/27011; Atwell et al., 1997; Carter, 2001): hierzu sollten spezifisch die schweren Ketten zwei verschiedener Antikörper einer unterschiedlichen Epitop-Spezifität miteinander paaren, indem sterisch in Kontakt tretende Aminosäure-Seitenketten an der Kontaktstelle der beiden Ketten in ihrer sterischen Raumverteilung invertiert werden: eine im Wildtyp bestehende kleine Aminosäure, die mit einer Aminosäure mit einer großen Seitenkette der anderen Kette interagiert, wird zu einer Aminosäure mit einer großen Seitenkette gentechnisch mutiert, wohingegen der große Aminosäure-Partner zu einem kleinen Rest getauscht wird (im Engl. "knob-hole" - Modell). Im Fall der Kettenpaarung beider mutierten Ketten treffen wiederum eine kleine auf eine große Aminosäure aufeinander, allerdings mit sterischer Inversion. Sollte das direkte Umfeld der Punktmutanten diesen sterischen Austausch zulassen, sollte die eigentliche Funktionalität des heterodimeren Moleküls (sprich die Epitop-Erkennung) nicht beeinträchtigt sein. Die WO 96/27011 beschreibt bispezifische Antikörper, Immunadhäsine oder Chimären davon. Dieser Ansatz läßt aber vollkommen die zusätzliche Steuerung der Interaktion von Oberflächen durch die Einführung von Ladungsgegensätzen außer acht, ist also lediglich ein 1-parametrisches Konzept. In dem hier beschriebenen Ansatz wird ein streng kombinatorisches, 2-parametrisches Konzept vorgestellt, das sowohl die Sterik als auch die Ladung berücksichtigt und damit impliziert, daß ein 1-parametrischer Ansatz in vielen Problemstellungen nicht vergleichbar effektiv die Kettenpaarung im gewünschten Sinne manipuliert. Das "knob hole"- Modell und das hier als die "kompensatorische Einführung von exponierten Ladungsträgern" beschriebene Modell sind daher als unabhängige Strategien zu erachten. Das letztere Modell läßt auch deutlich mehr Spielraum bei der Wahl der einzuführenden Mutationen zu und damit ein breiteres Spektrum an Lösungsmöglichkeiten.

Atwell et al. versuchten, bispezifische Antikörper herzustellen, in dem sie die zwei Hälften Epitop-differenter Antikörper an ihren Kontakstellen (CH₃-Domäne) der schweren Ketten miteinander verknüpfen (über "knob-hole"). Dabei bestehen wesentliche Unterschiede zu der vorliegenden Erfindung, die bei einem Transfer des Prinzips von Antikörpern auf TZR zu berücksichtigen sind. Ein Antikörper ist bivalent im Gegensatz zum monovalenten TZR. Das Resultat ist ein strukturell heterodimerer Antikörper, der zwei verschiedene Epitope erkennt (bispezifisch). Der erfindungsgemäße Ansatz geht in eine andere Richtung: hier wird versucht, die zwei predestinierten Ketten eines monospezifischen TZRs gezielt zu verknüpfen, damit eben keine funktionell heterodimere TZR mit potentiellen, von der gewünschten Monospezifität abweichenden Monospezifitäten entstehen.

Die Auswahl der auszutauschenden Aminosäuren erfolgte erfindungsgemäß durch "rationales Design": hierzu wurden bestehende TZR-Kristallstrukturen mittels Struktur-darstellender Software studiert und Aminosäure-Kandidaten zwecks punktueller Mutagenese bestimmt. Die Qualität der Aminosäureaustausche (den natürlichen Aminosäuresatz von 20 codogenen Aminosäuren in Säugern antizipierend) wurde je nach vorliegenden sterischen Gegebenheiten jeweils interagierender Aminosäurepaare unter Berücksichtigung des Umfeldes direkt benachbarter Aminosäuren individuell beurteilt. Das Modell-System, das etabliert wurde, um den Einfluß der Punktmutanten zu studieren, wird unten genauer beschrieben.

Das erfindungsgemäße Verfahren zur rationellen Mutagenese von TZR wird somit durch die oben genannten Publikationen sowie in der übrigen Literatur weder erwähnt noch nahegelegt. Die erfindungsgemäß eingeführten Punktmutationen je Kette sollen bewirken, daß präferentiell die von außen zugeführten TZR-Ketten paaren und nicht gemischte Pärchen mit den endogenen Ketten der T-Zellen ausbilden. Dies ist ein erheblicher Beitrag zum Thema Spezifität und damit Sicherheit der T-Zell-Antwort.

Im Unterschied zum Stand der Technik ist zu betonen, daß Punktmutanten deutlich geringer immunogen wirken als eingefügte, mehrere Aminosäuren umfassende Affinitäts-Anhängsel oder "Linker", wie es z.B. das Einzelketten-TZR - Konzept vorsieht. Zudem sind die Punktmutanten nahezu identisch zu den Wildtyp-Ketten, die bisher die größte funktionelle Wirksamkeit haben. Alle bisher und künftig entwickelten TAA-spezifischen TZR, ob murinen oder wahrscheinlich auch humanen Ursprungs, die künftig in der adoptiven Immuntherapie durch Gentransfer in humane T-Zellen von Tumorpatienten verwendet werden sollen, können mit geringem Aufwand mit diesen Mutationen ausgestattet werden. Der vorgestellte Ansatz könnte somit eine weite Verbreitung in der klinischen Applikation finden (Bolhuis et al., 1998; Cavazzana-Calvo et al., 2000).

In einer Variante des erfindungsgemäßen Verfahrens wird die nach der Mutagenese der DNA-Moleküle zunächst eingeführte Aminosäure weiter geeignet chemisch modifiziert. Weitere Mutationen können auch nicht-chemisch eingeführt werden, so zum Beispiel durch gentechnisch hergestellte Punktmutationen aus "phage display", um dadurch eine räumlich hervorstehende Gruppe oder eine räumlich zurückgesetzte Gruppe einzuführen. Dies bedeutet, daß zunächst eine Aminosäure eingeführt wird, die dann als Ausgangsbasis für die letztendlich vorhandene vorstehende Gruppe dient. Geeignete Modifikationen sind somit Aminosäurederivate, die durch chemische Mittel verändert wurden, wie zum Beispiel Methylierung (z. B. α-Methylvalin), Amidierung, insbesondere der C-terminalen Aminosäure durch ein Alkylamin (z. B. Ethylamin, Ethanolamin und Ethylendiamin) und Veränderungen einer Aminosäureseitenkette, wie zum Beispiel Acylierung der ε-Aminogruppe von Lysin. Andere Aminosäuren, die in die Kette inkorporiert werden können, schließen jede der D-Aminosäuren ein, die den 20 L-Aminosäuren, die gewöhnlich in Proteinen gefunden werden, entsprechen oder Iminioaminosäuren, seltene Aminosäuren, wie zum Beispiel Hydroxylysin oder nicht-Proteinaminosäuren, wie zum Beispiel Homoserin und Ornithin. Eine modifizierte Kette kann eines oder mehrerer dieser Derivate und D-Aminosäuren aufweisen. Die Kette kann durch chemische Verfahren synthetisiert werden, insbesondere unter der Verwendung eines automatisierten Peptid-Synthesizers oder durch rekombinante Verfahren hergestellt werden. Modifikationen des C-Terminus schließen Esterifizierung und Lactonbildung ein. N-terminale Modifikationen schließen Acetylierung, Acylierung, Alkylierung, Pegylierung, Myristylierung und ähnliches ein.

Üblicherweise werden jedoch mit dem erfindungsgemäßen Verfahren durch die nach der Mutagenese der DNA-Moleküle eingeführten Aminosäuren die räumlich hervorstehenden, bevorzugt geladenen und/oder polaren Gruppen oder die räumlich zurückgesetzten, bevorzugt entgegengesetzt geladenen und/oder polaren Gruppen direkt zur Verfügung gestellt, ohne daß eine weitere Modifikation erforderlich ist. Ein erfindungsgemäß besonders bevorzugtes Mutageneseverfahren bewirkt einen Austausch der Aminosäuren der ersten mit der der zweiten Kette oder umgekehrt, wobei die durch die Mutagenese der DNA-Moleküle eingeführten Aminosäuren so gewählt sind, daß ein wechselseitiger Austausch der Aminosäuren der an den Oberflächen der wechselwirkenden Ketten des TZR erreicht wird.

Als räumlich zurückgesetzte, bevorzugt geladene und/oder polare Gruppe soll im Sinne der vorliegenden Erfindung jede chemisch an der jeweils zu mutierenden Kette angebrachte chemische Gruppe verstanden werden, die räumlich weniger Platz einnimmt, als deren entsprechende vorher anwesende Gruppe und bevorzugt eine ganze bzw. den Bruchteil einer ganzen Ladungseinheit trägt. Es wird eine zur räumlich vorstehenden geladenen und/oder polaren Gruppe entgegengesetzte Ladung eingeführt, entweder in der zurückversetzten Gruppe selbst durch Einführung einer Ladung oder aber durch den Wegfall einer Ladung in der betreffenden Gruppe wird die Nettoladung des die räumlich vorstehende geladene und/oder polare Gruppe umgebenden Käfigs umgekehrt (Figur 11). So kann die nach der Mutagenese der DNA-Moleküle eingeführte Aminosäure, die gegenüber der Ausgangssequenz eine räumlich zurückgesetzte Gruppe einführt ausgewählt sein aus Glyzin, Serin, Threonin, Alanin, ohne darauf begrenzt zu sein. Serin und Threonin sind hierbei diejenigen zurückversetzten Gruppen, die selbst eine Partialladung tragen. Die Verwendung von Glyzin und Alanin impliziert die Betonung der verbleibenden Nettoladung des umgebenden Käfigs (z.B. Partialladungen der Peptidbindungen im Protein-Rückgrat). Ähnlich soll als räumlich vorstehende Gruppe im Sinne der vorliegenden Erfindung jede chemisch an der jeweils zu mutierenden Kette angebrachte chemische Gruppe verstanden werden, die räumlich mehr Platz einnimmt, als deren entsprechende vorher anwesende Gruppe und bevorzugt eine ganze bzw. den Bruchteil einer ganzen Ladungseinheit trägt. So kann die nach der Mutagenese der DNA-Moleküle eingeführte Aminosäure, die gegenüber der Ausgangssequenz eine räumlich vorstehende Gruppe einführt, ausgewählt sein aus Lysin, Arginin, Histidin, Cystein, Glutamin, Glutamat und Tyrosin, ohne darauf beschränkt zu sein.

In einem weiteren Aspekt der vorliegenden Erfindung werden in einem erfindungsgemäßen Verfahren mindestens zwei Oberflächen einer TZR-Kette gleichzeitig einer Mutagenese unterzogen. Dadurch kann mittels der Akkumulierung von mehreren Mutationen unter Umständen eine weiter gesteuerte Paarungseigenschaft des entsprechenden TZR erreicht werden, so zum Beispiel eine energetisch stärkere oder weniger starke Paarungskinetik, verglichen mit dem nicht modifizierten Zustand. Ein Verfahren zur Optimierung von Wechselwirkungen mehrerer Oberflächen stellt das "phage-display"-Verfahren dar.

Gemäß einem weiteren erfindungsgemäßen Verfahren können die entsprechend in Wechselwirkung stehenden Oberflächen in den variablen Domänen der TZR-Ketten lokalisiert sein. Die entsprechend in Wechselwirkung stehenden Oberflächen können jedoch auch in den konstanten Domänen der TZR-Ketten lokalisiert sein. Die Lokalisierung dieser Modifikationen hängt u.a. von der erwünschten Spezifizierung des jeweiligen TZR und dessen spezifischen Interaktionen ab.

So können gemäß einem besonderen erfindungsgemäßen Verfahren die zu mutierenden Domänen der TZR-Ketten ausgewählt sein aus Säugetier-, und dabei insbesondere aus menschlichen und/oder Maus-Domänen. Besonders bevorzugt ist dabei eine Mutagenese gemäß der vorliegenden Erfindung, wobei die rationale Mutagenese der TZR-Ketten gleichzeitig zu einer Humanisierung des TZR führt. Diese Mutagenese führt somit u.a. zu einer verbesserten Verträglichkeit des TZR.

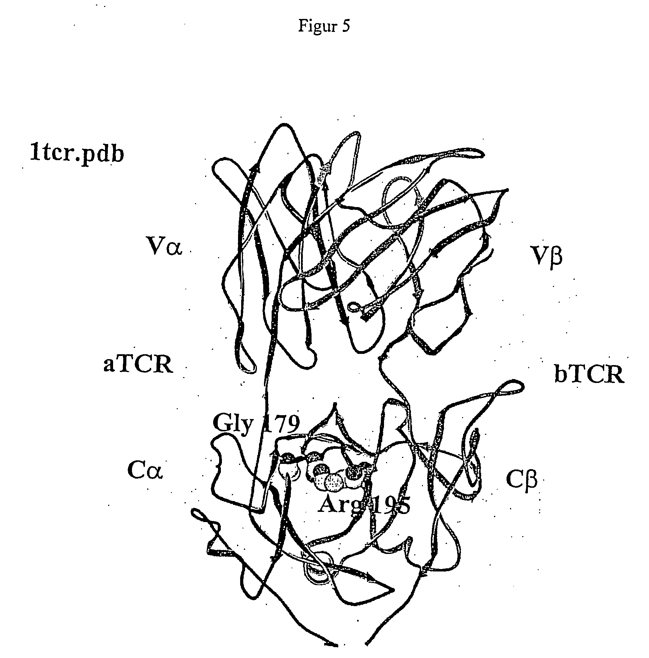

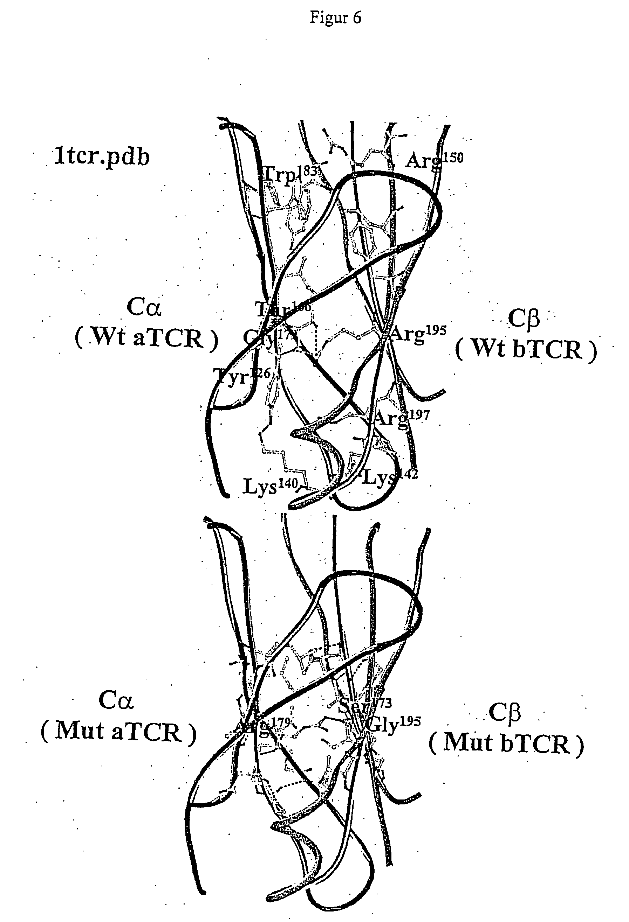

Die vorliegende Erfindung weist somit im wesentlichen drei Komponenten auf:
a) Der vom "knob hole" - Modell unabhängige Ansatz über große sterische Gruppen Ladung in das Innere der komplementären Kette einzubringen und durch kleine sterische Gruppen selbst oder durch den Wegfall geladener Gruppen zu einer Ladungskompensierung der eingebrachten Ladung zu gelangen. Dies ist ein strikt 2-parametrischer Ansatz. Hierbei wurde erstmals versucht, dieses Modell auf die Kontaktfläche zwischen den heterodimeren Ketten eines monovalenten TZR anzuwenden, wohingegen bisher bei Antikörpern diese auf die Kontaktfläche zwischen den schweren Ketten eines bivalent strukturierten Antikörpers bezogen wurde.
b) Das experimentell neue an der vorliegenden Erfindung ist, ein Aminosäurepaar im TZR zu bestimmen, für das das beschriebene Modell anwendbar ist (αTZR/Gly¹⁹² und βTZR/Arg²⁰⁸ des MDM2(81-88)-spezifischen TZR, Figur 5) und die Qualität des Austausches festzulegen (^{Arg}208^{Gly} und ^{Gly}192^{Arg}). Es handelt sich um eine 1:1 Inversion der vorgefundenen sterischen und Ladungs-abhängigen Gegebenheiten und entspricht dem Übergang von Zustand I (Wildtyp) zu Zustand II (1 Mutation/Kette) der Figur 11 bzw. 2: Die eingebrachte positive Ladung des Arginin wird durch die verbliebenen Carbonyle des Polypeptidfadens der komplemtären Kette kompensiert. Ein weiter Indikator für eine zum Wildtyp vergleichbare Ladungsneutralisation ist die durch Proteinstrukturen-Datenbank unterstützte Modellierbarkeit von Wasserstoffbrücken zwischen der Guanidinium-Gruppe des Arginin und den Hauptketten-Carbonylen sowohl für Zustand I als auch II für die Kristallstruktur eines ebenfalls murinen TZR (1tcr.pdb der Figur6).
c) Das hier beispielhaft zu bestimmende Aminosäurepaar sollte so gewählt werden, daß es zum einen auf alle murinen TZR zu verallgemeinern war, um künftige, für andere TAA-spezifische murine TZR ebenso mit diesen Mutationen ausstatten zu können, und zum anderen auch auf humane TZR erweitert werden konnte. Es wurde die konstante Domäne gewählt, da hier hinreichende Homologie zwischen humanen und murinen TZR besteht, um davon auszugehen, daß die gewählten Mutationen einen vergleichbar detrimentalen Effekt für hybride Hu/Mu TZR haben, wie es in der vorliegenden Erfindung für murine "hybride" TZR stellvertretend gezeigt wurde.

Es ist zu berücksichtigen, daß durchaus andere Aminosäureaustausche an der betreffenden Position einen vergleichbaren Effekt aufweisen könnten. Ferner ist eine Optimierung durch weitere, kumulative Austausche in der Umgebung der betreffenden Punktmutationen denkbar.

Ein weiterer Aspekt der vorliegenden Erfindung betrifft ein Verfahren, wobei als alpha-Kette und beta-Kette die alpha- und beta-Ketten eines MDM2(81-88)-spezifischen TZR verwendet werden und wobei das Gly₁₉₂ der konstanten Region der alpha-Kette und das Arg²⁰⁸ der konstanten Region der beta-Kette gegen Arg₁₉₂ in der konstanten Region der alpha-Kette und gegen Gly₂₀₈ in der konstanten Region der beta-Kette ausgetauscht werden. Anhand dieses TZR konnte das erfindungsgemäße Prinzip erstmals erfolgreich angewendet werden.

Bevorzugt ist weiterhin ein erfindungsgemäßes Verfahren, wobei als Transfektionssystem ein retroviraler Vektor, insbesondere pBullet verwendet wird. In den Vektoren können auch IRES-Elemente verwendet werden.

Ein weiterer Aspekt der vorliegenden Erfindung betrifft eine mutierte erste (alpha-) oder zweite (beta-) Kette eines TZR, die nach einem Verfahren gemäß der vorliegenden Erfindung hergestellt wird. Weiter besonders bevorzugt ist ein erfindungsgemäß mutierter TZR, insbesondere ein mutierter MDM2(81-88)-spezifischer TZR, wobei dieser mindestens eine mutierte alpha- und beta-Kette aufweist. Dieser mutierte TZR gemäß der vorliegenden Erfindung kann auch in Form eines Fusionsproteins, umfassend die erfindungsgemäß modifizierten Polypeptide oder Teile davon vorliegen. Das Fusionsprotein kann dadurch gekennzeichnet sein, daß es die ζ-Region von CD3 oder CD8 oder CD 16 oder Teile davon umfaßt, insbesondere die ζ-Region von humanem CD3 oder CD8 oder CD16 oder Teile davon. Insbesondere kann das erfindungsgemäße Fusionsprotein die ζ-Kette des CD3-Komplexes oder ITAM-Motive der ζ-Kette oder Teile davon umfassen, insbesondere die ζ-Kette von humanem CD3 oder Teile davon. Das Fusionsprotein kann weiterhin dadurch gekennzeichnet sein, daß es CD8α oder das Lck-Bindungsmotiv von CD8α umfaßt oder Teile davon, insbesondere von humanem CD8α.

Ein weiterer Aspekt der Erfindung betrifft eine isolierte Nukleinsäure, die eine für eine mutierte erste (z.B. alpha-) oder zweite (z.B. beta-) Kette eines erfindungsgemäßen TZR kodierende Sequenz umfaßt. Diese erfindungsgemäße Nukleinsäure kann eine DNA, RNA, PNA (Peptide nucleic acid) oder p-NA (Pyranosyl nucleic acid), vorzugsweise eine DNA, insbesondere eine doppelsträngige DNA mit einer Länge von mindestens 8 Nukleotiden, vorzugsweise mit mindestens 18 Nukleotiden, insbesondere mit mindestens 24 Nukleotiden sein. Die Nukleinsäure kann dadurch gekennzeichnet sein, daß die Sequenz der Nukleinsäure mindestens ein Intron und/oder eine polyA-Sequenz aufweist. Sie kann auch in Form ihrer antisense-Sequenz vorliegen.

Ein weiterer Aspekt der Erfindung betrifft auch ein DNA- oder RNA-Vektormolekül, das mindestens eine oder mehrere erfindungsgemäße Nukleinsäure(n) umfaßt und das in Zellen exprimierbar ist. Für die Expression des betreffenden Gens ist im allgemeinen eine doppelsträngige DNA bevorzugt, wobei der für das Polypeptid kodierende DNA-Bereich besonders bevorzugt ist. Dieser Bereich beginnt mit dem ersten in einer Kozak Konsensus Sequenz (Kozak, 1987, Nucleic. Acids Res. 15:8125-48) liegenden Start-Codon (ATG) bis zum nächsten Stop-Codon (TAG, TGA bzw. TAA), das im gleichen Leseraster zum ATG liegt. Eine weitere Verwendung der erfindungsgemäßen Nukleinsäuresequenzen ist die Konstruktion von anti-sense Oligonukleotiden (Zheng und Kemeny, 1995, Clin. Exp. Immunol. 100:380-2) und/oder Ribozymen (Amarzguioui, et al. 1998, Cell. Mol. Life Sci. 54:1175-202; Vaish, et al., 1998, Nucleic Acids Res. 26:5237-42; Persidis, 1997, Nat. Biotechnol. 15:921-2). Mit anti-sense Oligonukleotiden kann man die Stabilität der erfindungsgemäßen Nukleinsäure verringern und/oder die Translation der erfindungsgemäßen Nukleinsäure inhibieren. So kann beispielsweise die Expression der entsprechenden Gene in Zellen sowohl in vivo als auch in vitro verringert werden. Oligonukleotide können sich daher als Therapeutikum eignen. Diese Strategie eignet sich beispielsweise auch für Haut, epidermale und dermale Zellen, insbesondere, wenn die antisense Oligonukleotide mit Liposomen komplexiert werden (Smyth et al., 1997, J. Invest. Dermatol. 108:523-6; White et al., 1999, J. Invest. Dermatol. 112:699-705). Für die Verwendung als Sonde oder als "antisense" Oligonukleotid ist eine einzelsträngige DNA oder RNA bevorzugt.

Neben den aus Zellen isolierten natürliche Nukleinsäuren können alle erfindungsgemäßen Nukleinsäuren oder deren Teile auch synthetisch hergestellt worden sein. Weiterhin kann zur Durchführung der Erfindung eine Nukleinsäure verwendet werden, die synthetisch hergestellt worden ist. So kann die erfindungsgemäße Nukleinsäure beispielsweise chemisch anhand der beschriebenen Proteinsequenzen unter Heranziehen des genetischen Codes z. B. nach der Phosphotriester-Methode synthetisiert werden (siehe z. B. Uhlmann, E. & Peyman, A. (1990) Chemical Revievs, 90, 543-584).

Oligonukleotide werden in der Regel schnell durch Endo- oder Exonukleasen, insbesondere durch in der Zelle vorkommende DNasen und RNasen, abgebaut. Deshalb ist es vorteilhaft, die Nukleinsäure zu modifizieren, um sie gegen den Abbau zu stabilisieren, so daß über einen langen Zeitraum eine hohe Konzentration der Nukleinsäure in der Zelle beibehalten wird (WO 95/11910; Macadam et al., 1998, WO 98/37240; Reese et al., 1997, WO 97/29116). Typischerweise kann eine solche Stabilisierung durch die Einführung von einer oder mehrerer Internukleotid-Phosphorgruppen oder durch die Einführung einer oder mehrerer Nicht-Phosphor-Internukleotide, erhalten werden.

Geeignete modifizierte Internukleotide sind in Uhlmann und Peymann (1990 Chem. Rev. 90, 544) zusammengefaßt (WO 95/11910; Macadam et al., 1998, WO 98/37240; Reese et al., 1997, WO 97/29116). Modifizierte Internukleotid-Phosphatreste und/oder Nicht-Phosphorbrücken in einer Nukleinsäure, die bei einer der erfindungsgemäßen Verwendungen eingesetzt werden können, enthalten zum Beispiel Methylphosphonat, Phosphorothioat, Phosphoramidat, Phosphorodithioat, Phophatester, während Nicht-Phosphor-Internukleotid-Analoge, beispielsweise Siloxanbrücken, Carbonatbrücken, Carboxymethylester, Acetamidatbrücken und/oder Thioetherbrücken enthalten. Es ist auch beabsichtigt, daß diese Modifizierung die Haltbarkeit einer pharmazeutischen Zusammensetzung, die bei einer der erfindungsgemäßen Verwendungen eingesetzt werden kann, verbessert.

Ein weiterer Aspekt der vorliegenden Erfindung betrifft einen Vektor, vorzugsweise in Form eines Plasmids, shuttle Vektors, Phagemids, Cosmids, Expressionsvektors, adenoviralen Vektors, retroviralen Vektors (Miller, et al. "Improved retroviral vectors for gene transfer and expression", BioTechniques Vol. 7, No. 9, p 980, 1989) und/oder gentherapeutisch wirksamen Vektors, der eine erfindungsgemäße Nukleinsäure enthält.

So kann die erfindungsgemäße Nukleinsäure in einem Vektor vorzugsweise in einem Expressionsvektor oder gentherapeutisch wirksamen Vektor enthalten sein. Vorzugsweise enthält der gentherapeutisch wirksame Vektor T-Zell spezifische regulatorische Sequenzen, die funktionell mit der erfindungsgemäßen Nukleinsäure verbunden sind. Die Expressionsvektoren können prokaryotische oder eukaryotische Expressionsvektoren sein. Beispiele für prokaryotische Expressionsvektoren sind für die Expression in *E*. *coli* z.B. die Vektoren pGEM oder pUC-Derivate und für eukaryotische Expressionsvektoren für die Expression in *Saccharomyces cerevisiae* z. B. die Vektoren p426Met25 oder p426GAL1 (Mumberg et al. (1994) Nucl. Acids Res., 22, 5767-5768), für die Expression in Insektenzellen z. B. *Baculovirus*-Vektoren wie in EP-B1-0 127 839 oder EP-B1-0 549 721 offenbart, und für die Expression in Säugerzellen z. B. die Vektoren Rc/CMV und Rc/RSV oder SV40-Vektoren, welche alle allgemein erhältlich sind.

Im allgemeinen enthalten die Expressionsvektoren auch für die jeweilige Wirtszelle geeignete Promotoren, wie z. B. den trp-Promotor für die Expression in E. coli (siehe z. B. EP-B1-0 154 133), den Met 25, GAL 1 oder ADH2-Promotor für die Expression in Hefen (Russel et al. (1983), J. Biol. Chem. 258, 2674-2682; Mumberg, supra), den Baculovirus-Polyhedrin-Promotor für die Expression in Insektenzellen (siehe z. 13. EP-B1-0 127 839). Für die Expression in Säugetierzellen sind beispielsweise Promotoren geeignet, die eine konstitutive, regulierbare, gewebsspezifische, zellzyklusspezifische oder metabolischspezifische Expression in eukaryotischen Zellen erlauben. Regulierbare Elemente gemäß der vorliegenden Erfindung sind Promotoren, Aktivatorsequenzen, Enhancer, Silencer und/oder Repressorsequenzen. Beispiel für geeignete regulierbare Elemente, die konstitutive Expression in Eukaryonten ermöglichen, sind Promotoren, die von der RNA Polymerase III erkannt werden oder virale Promotoren, CMV-Enhancer, CMV-Promotor, CMV-LTR-Hybride, SV40 Promotor oder LTR-Promotoren z. B. von MMTV (mouse mammary tumour virus; Lee et al. (1981) Nature 214, 228-232) und weitere virale Promotor- und Aktivatorsequenzen, abgeleitet aus beispielsweise HBV, HCV, HSV, HPV, EBV, HTLV oder HIV. Ein Beispiel für ein regulierbares Element, das eine regulierbare Expression in Eukaryonten ermöglicht, ist der Tetracyclinoperator in Kombination mit einem entsprechenden Repressor (Gossen M. et al. (1994) Curr. Opin. Biotechnol. 5, 516-20).

Beispiele für regulierbare Elemente, die T-Zell spezifische Expression in Eukaryonten ermöglichen, sind Promotoren oder Aktivatorsequenzen aus Promotoren oder Enhancern von solchen Genen, die für Proteine kodieren, die nur in diesen Zelltypen exprimiert werden.

Beispiele für regulierbare Elemente, die Zellzyklus-spezifische Expression in Eukaryonten ermöglichen, sind Promotoren folgender Gene: cdc25, Cyclin A, Cyclin E, cdc2, E2F, B-myb oder DHFR, (Zwicker J. und Müller R. (1997) Trends Genet. 13, 3-6). Beispiele für regulierbare Elemente, die metabolischspezifische Expression in Eukaryonten ermöglichen, sind Promotoren, die durch Hypoxie, durch Glukosemangel, durch Phosphatkonzentration oder durch Hitzeschock reguliert werden.

Der erfindungsgemäße Vektor kann zur Transfektion einer Wirtszelle verwendet werden, bei der es sich bevorzugterweise um eine T-Zelle handelt. Besonders bevorzugt ist eine Wirtszelle, die dadurch gekennzeichnet ist, daß sie auf ihrer Oberfläche ein erfindungsgemäßes Polypeptid oder Fusionsprotein exprimiert. Ein weiterer Gegenstand der Erfindung betrifft somit ein Verfahren zur Herstellung eines Polypeptids zur Diagnose und/oder Behandlung von mit Onkoproteinen in Zusammenhang stehenden Erkrankungen oder zur Identifizierung von pharmakologisch aktiven Substanzen in einer geeigneten Wirtszelle, das dadurch gekennzeichnet ist, daß eine erfindungsgemäße Nukleinsäure auf geeignete Weise exprimiert wird.

Das Polypeptid wird so beispielsweise durch Expression der erfindungsgemäßen Nukleinsäure in einem geeigneten Expressionssystem, wie oben bereits beschrieben, nach dem Fachmann allgemein bekannten Methoden hergestellt. Als Wirtszellen eignen sich beispielsweise die *E. coli* Stämme DHS, HB101 oder BL21, der Hefestamm *Saccharomyces cerevisiae,* Insektenzellinien, z. B. von *Spodoptera frugiperda,* oder die tierischen Zellen COS, Vero, 293, HaCaT, und HeLa, die alle allgemein erhältlich sind.

Um die Einführung von erfindungsgemäßen Nukleinsäuren und damit die Expression des Polypeptids in einer eu- oder prokaryotischen Zelle durch Transfektion, Transduktion, Transformation oder Infektion zu ermöglichen, kann die Nukleinsäure als Plasmid, als Teil eines viralen oder nicht-viralen Vektors oder Partikels vorliegen. Als virale Vektoren oder Partikel eignen sich hierbei besonders: Baculoviren, Vakziniaviren, Retroviren, Adenoviren, adenoassoziierte Viren und Herpesviren. Als nicht-virale Träger eignen sich hierbei besonders: Virosomen, Liposomen, kationische Lipide, oder poly-Lysin konjugierte DNA.

Beispiele von gentherapeutisch wirksamen Vektoren sind Virusvektoren, beispielsweise Adenovirusvektoren oder retrovirale Vektoren (Lindemann et al., 1997, Mol. Med. 3: 466-76; Springer et al., 1998, Mol. Cell. 2: 549-58; Weijtens et al. "A retroviral vector system ,'STICH'; in combination with an optimized single chain antibody chimeric receptor gene structure allows efficient gene transduction and expression in human T-lymphocytes", Gene Therapy (1998) 5,1995-1203).

Ein bevorzugter Mechanismus, erfindungsgemäße Polypeptide *in vivo* zur Expression zu bringen, ist der virale Gen-Transfer, insbesondere mit Hilfe retroviraler Partikel. Diese werden vorzugsweise genutzt, entsprechende Zielzellen, vorzugsweise T-Lymphozyten, des Patienten *ex vivo* mit den für erfindungsgemäße Polypeptide kodierenden Genen oder Nukleotidsequenzen durch Transduktion zu versehen. Die Zielzellen können daraufhin im Sinne eines adoptiven Zelltransfers wieder in den Patienten reinfundiert werden, um mit der *de novo* eingefügten Spezifität tumorizide und/oder immunmodulierende Effektorfunktionen zu übernehmen. Jüngst wurden auf diesem Wege sehr gute gentherapeutische Erfolge in der Behandlung der durch Immuninkompetenz gekennzeichneten SCID-X1- Krankheit bei Neugeborenen erzielt, in dem hämatologische Vorläuferzellen mit einem analogen intakten Transgen einer in den Kindern vorkommenden nicht-funktionellen mutierten Variante des γ-Kettengens, das für die Differenzierung in die verschiedenen Effektorzellen des adaptiven Immunsystems essentiell ist, retroviral versehen wurden (Cavazzana-Calvo et al., 2000).

Weiterhin besteht die Möglichkeit den Gentransfer *in vivo* durchzuführen, einerseits durch präferentiell stereotaktische Injektion der infektiösen Partikel, andererseits durch direkte Applikation von Viren-produzierenden Zellen (Oldfield, et al. Hum. Gen. Ther., 1993, 4:39-69).

Die zum Transfer von Genen häufig eingesetzten viralen Vektoren sind nach heutigem Stand der Technik vorwiegend retrovirale, lentivirale, adenovirale und adeno-assoziierte virale Vektoren. Diese sind von natürlichen Viren abgeleitete zirkuläre Nukleotidsequenzen, in denen zumindest die viralen Strukturprotein-kodierenden Gene durch das zu transferierende Konstrukt ausgetauscht werden.

Retrovirale Vektorsysteme schaffen die Voraussetzung für eine langhaltende Expression des Transgens durch die stabile, aber ungerichtete Integration in das Wirtsgenom. Vektoren der jüngeren Generation besitzen keine irrelevanten und potentiell immunogenen Proteine, des weiteren gibt es keine vorbestehende Immunität des Empfängers gegenüber dem Vektor. Retroviren enthalten ein RNA-Genom, das in eine Lipidhülle verpackt ist, die aus Teilen der Wirtszellmembran und Virusproteinen besteht. Zur Expression viraler Gene wird das RNS-Genom revers transkribiert und mit dem Enzym Integrase in die Zielzell-DNS integriert. Diese kann daraufhin von der infizierten Zelle transkribiert und translatiert werden, wodurch virale Bestandteile entstehen, die sich zu Retroviren zusammenfügen. RNS wird ausschließlich dann in die neu entstandenen Viren eingefügt. Das Genom der Retroviren besitzt drei essentielle Gene: *gag,* das für virale Strukturproteine, sogenannte gruppenspezifische Antigene kodiert, *pol* für Enzyme wie Reverse Transkriptase und Integrase und *env* für das Hüllprotein ("envelope"), das für die Bindung des wirtsspezifischen Rezeptors verantwortlich ist. Die Produktion der replikationsinkompetenten Viren findet nach Transfektion in sogenannten Verpackungszelllinien statt, die zusätzlich mit den gag/polkodierenden Genen ausgestattet wurden und diese "in trans" exprimieren und somit die Ausbildung replikationsinkompetenter (d.h. gag/pol-deletierter) transgener Viruspartikel komplementieren. Eine Alternative ist die Cotransfektion der essentiellen Virusgene, wobei nur der das Transgen enthaltende Vektor das Verpackungssignal trägt.

Die Separation dieser Gene ermöglicht einerseits die beliebige Kombination des gal/pol-Leserahmens mit aus verschiedenen Stämmen gewonnenen *env*-Leserahmen, wodurch Pseudotypen mit verändertem Wirtstropismus entstehen, andererseits kann dadurch die Bildung replikationskompetenter Viren innerhalb von Verpackungszellen drastisch reduziert werden. Das aus "gibbon ape leukemia virus" (GALV) abgeleitete Hüllprotein, das im "stitch" bzw. "bullet"-Vektorsystem Verwendung findet, ist in der Lage humane Zellen zu transduzieren und ist in der Verpackungszelllinie PG13 mit amphotropen Wirtsbereich etabliert (Miller et al., 1991). Zusätzlich wird die Sicherheit durch selektive Deletion von nicht-essentiellen Virussequenzen zur Verhinderung einer homologen Rekombination und somit der Produktion replikationskompetenter Partikel erhöht.

Neue, nicht-virale Vektoren bestehen aus autonomen, sich selbst-integrierenden DNS-Sequenzen, den Transposonen, die durch z.B. liposomale Transfektion in die Wirtszelle eingeschleußt werden und erstmals erfolgreich zur Expression humaner Transgene in Säugerzellen eingesetzt wurden (Yant et al., 2000).

Gentherapeutisch wirksame Vektoren lassen sich auch dadurch erhalten, daß man die erfindungsgemäße Nukleinsäure mit Liposomen komplexiert, da damit eine sehr hohe Transfektionseffizienz, insbesondere von Hautzellen, erreicht werden kann (Alexander und Akhurst, 1995, Hum. Mol. Genet. 4: 2279-85). Hilfsstoffe, die den Transfer von Nukleinsäuren in die Zelle erhöhen, können beispielsweise Proteine oder Peptide, die an DNA gebunden sind oder synthetische Peptid-DNA-Moleküle, die den Transport der Nukleinsäure in den Kern der Zelle ermöglichen, sein (Schwartz et al. (1999) Gene Therapy 6, 282; Branden et al. (1999) Nature Biotech. 17, 784). Hilfsstoffe umfassen auch Moleküle, die die Freisetzung von Nukleinsäuren in das Cytoplasma der Zelle ermöglichen (Planck et al. (1994) J. Biol. Chem. 269, 12918; Kichler et al. (1997) Bioconj. Chem. 8, 213) oder beispielsweise Liposomen (Uhlmann und Peymann (1990) supra). Eine andere besonders geeignete Form von gentherapeutischen Vektoren läßt sich dadurch erhalten, daß man die erfindungsgemäße Nukleinsäure auf Goldpartikeln aufbringt und diese mit Hilfe der sogenannten "Gene Gun" in Gewebe, bevorzugt in die Haut, oder Zellen schießt (Wang et al., 1999, J. Invest. Dermatol., 112:775-81).

Für die gentherapeutische Anwendung der erfindungsgemäßen Nukleinsäure ist es auch von Vorteil, wenn der Teil der Nukleinsäure, der für das Polypeptid kodiert, ein oder mehrere nicht kodierende Sequenzen einschließlich Intronsequenzen, vorzugsweise zwischen Promotor und dem Startcodon des Polypeptids, und/oder eine polyA-Sequenz, insbesondere die natürlich vorkommende polyA-Sequenz oder eine SV40 Virus polyA-Sequenz, vor allem am 3'-Ende des Gens enthält, da hierdurch eine Stabilisierung der mRNA erreicht werden kann (Jackson, R. J. (1993) Cell 74, 9-14 und Palmiter, R. D. et al. (1991) Proc. Natl. Acad. Sci. USA 88, 478-482).

Ein weiterer Aspekt der vorliegenden Erfindung betrifft eine Wirtszelle, die ein DNA- oder RNA-Vektormolekül gemäß der Erfindung enthält. Diese kann insbesondere eine T-Zelle sein, die mit einem erfindungsgemäßen Vektor oder einem anderen erfindungsgemäßen Genkonstrukt transformiert ist. Wirtszellen können sowohl prokaryotische als auch eukaryotische Zellen sein, Beispiele für prokaryotische Wirtszellen sind *E. coli* und für eukaryotische Zellen *Saccharomyces cerevisiae* oder Insektenzellen.

Ein weiterer Aspekt betrifft somit eine rekombinante T-Zelle, die mindestens einen mutierten TZR gemäß der vorliegenden Erfindung exprimiert. Eine besonders bevorzugte transformierte Wirtszelle ist eine transgene T-Vorläuferzelle oder eine Stammzelle, die dadurch gekennzeichnet ist, daß sie ein erfindungsgemäßes Genkonstrukt oder eine erfindungsgemäße Expressionskassette umfaßt. Verfahren zur Transformation oder Transduktion von Wirtszellen und/oder Stammzellen sind dem Fachmann gut bekannt und umfassen zum Beispiel Elektroporation oder Mikroinjektion. Eine besonders bevorzugte transformierte Wirtszelle ist eine patienteneigene T-Zelle, die nach der Entnahme mit einem erfindungsgemäßen Genkonstrukt transfiziert wird. Erfindungsgemäße Wirtszellen können insbesondere dadurch erhalten werden, daß dem Patienten eine oder mehrere Zellen, bevorzugterweise T-Zellen, insbesondere CD8⁺-T-Zellen entnommen werden, die dann *ex vivo* mit einem oder mehreren erfindungsgemäßen genetischen Konstrukten transfiziert oder transduziert werden, um so erfindungsgemäße Wirtszellen zu erhalten. Die *ex vivo* generierten spezifischen T-Zellen können dann anschließend in den Patienten reimplantiert werden. Das Verfahren ähnelt somit dem bei Darcy et al. ("Redirected perforin-dependent lysis of colon carcinoma by ex vivo genetically engineered CTL" J. Immunol., 2000, 164:3705-3712) beschriebenen Verfahren unter der Verwendung von scFv anti-CEA Rezeptor transduzierten ZTL, Perforin und γ-IFN.

Die erfindungsgemäß modifizierten (Poly)peptide und deren Derivate können zum Beispiel auch zur aktiven und/oder passiven Immunisierung von Patienten mit Erkrankungen, insbesondere Tumorerkrankungen, die zum Beispiel mit MDM2 in Zusammenhang stehen, eingesetzt werden. Ein besonders bevorzugter Aspekt der vorliegenden Erfindung betrifft somit eine Verwendung bei der eine Krebserkrankung behandelt wird, insbesondere eine Krebserkrankung, die mit einer veränderten Expression von MDM2 in Zusammenhang steht, um so die Induktion, Erzeugung und Zunahme vom Onkogen-spezifischen, z.B. MDM2-spezifischen ZTL zu erreichen und die Tumor- und Leukämiezellen der betreffenden Patienten spezifisch abzutöten. Solche Erkrankungen umfassen zum Beispiel solide Tumorerkrankungen, lymphohämatopoetische Neoplasien, maligne hämatologische Erkrankungen, auch in Form eines multiplen Myeloms (oder Plastozytoms), eines histiozytischen Lymphoms und eines CML-Blastenschubs. Damit zusamenhängende TAAs, gegen die entsprechende TZR entwickelt werden können, sind zum Beispiel p53, Her-2/neu, Ras, Tyrosinase, MART, Gp100, MAGE, BAGE, MUC-1, CD45, CD19 und PRDI-BF1.

Ein besonders bevorzugter Aspekt der vorliegenden Erfindung betrifft somit auch eine Zusammensetzung, insbesondere pharmazeutische Zusammensetzung, die eine rekombinante T-Zelle gemäß der vorliegenden Erfindung. Bevorzugt ist weiterhin die Verwendung einer mutierten alpha- oder beta-Kette eines TZR gemäß der vorliegenden Erfindung, eines mutierten TZR gemäß gemäß der vorliegenden Erfindung und/oder einer rekombinanten T-Zelle gemäß gemäß der vorliegenden Erfindung zur Herstellung von Therapeutika und/oder Prophylaktika zur Behandlung von Krebserkrankungen. Bei einer besonders bevorzugten Art der Behandlung wird dem Patienten eine oder mehrere Zellen, bevorzugterweise T-Zellen, insbesondere CD8⁺-T-Zellen entnommen, die dann ex vivo mit einem oder mehreren erfindungsgemäßen genetischen Konstrukten transduziert oder transfiziert werden. Die *ex vivo* generierten spezifischen T-Zellen können dann anschließend in den Patienten reimplantiert werden. Die erfindungsgemäße Zusammensetzung kann weiterhin geeignete Zusatz- und Hilfsstoffe enthalten.

Ein Gegenstand der vorliegenden Erfindung ist auch ein Arzneimittel zur Indikation und Therapie von mit Onkoprotein-Protein assoziierten Erkrankungen, das eine erfindungsgemäße Nukleinsäure oder ein erfindungsgemäßes Polypeptid und gegebenenfalls geeignete Zusatz- oder Hilfsstoffe enthält, sowie ein Verfahren zur Herstellung eines solchen Arzneimittels zur Behandlung von mit Onkoprotein-Protein assoziierten Erkrankungen, bei dem eine erfindungsgemäße Nukleinsäure oder ein erfindungsgemäßes Polypeptid mit einem pharmazeutisch annehmbaren Träger formuliert wird. Als Therapeutika und/oder Prophylaktika kommen insbesondere Impfstoffe, rekombinante Partikel oder Injektionen oder Infusionslösungen in Betracht, die als Wirkstoff (a) das erfindungsgemäße TZR-Rezeptor Polypeptid und/oder seine Derivate und/oder (b) eine erfindungsgemäße Nukleinsäure enthalten und/oder (c) *in vitro* oder *ex vivo* erzeugte T-Lymphozyten, die einen spezifisch mutierten gegen Onkoprotein gerichteten TZR enthalten.

Für die gentherapeutische Anwendung beim Menschen ist vor allem ein Arzneimittel und/oder rekombinanter Partikel geeignet, das die erfindungsgemäße Nukleinsäure in nackter Form oder in Form eines der oben beschriebenen gentherapeutisch wirksamen Vektoren oder in mit Liposomen bzw. Goldpartikeln komplexierter Form enthält. Der pharmazeutische Träger ist beispielsweise eine physiologische Pufferlösung, vorzugsweise mit einem pH von ca. 6,0-8,0, vorzugsweise von ca. 6,8-7,8. Insbesondere von ca. 7,4 und/oder einer Osmolarität von ca. 200-400 milliosmol/Liter, vorzugsweise von ca. 290-310 milliosmol/Liter. Zusätzlich kann der pharmazeutische Träger geeignete Stabilisatoren, wie z. B. Nukleaseinhibitoren, vorzugsweise Komplexbildner wie EDTA und/oder andere dem Fachmann bekannte Hilfsstoffe enthalten.

Der Begriff "kodierende Nukleinsäure" bezieht sich auf eine DNA-Sequenz, die für ein isolierbares bioaktives erfindungsgemäßes Polypeptid oder einen Vorläufer kodiert. Das Polypeptid kann durch eine Sequenz in voller Länge oder jeden Teil der kodierenden Sequenz kodiert werden, solange die spezifische, beispielsweise enzymatische Aktivität erhalten bleibt.

Es ist bekannt, daß kleine Veränderungen in der Sequenz der erfindungsgemäßen Nukleinsäuren vorhanden sein können, zum Beispiel durch die Degenerierung des genetischen Codes, oder daß nicht translatierte Sequenzen am 5' und/oder 3'-Ende der Nukleinsäure angehängt sein können, ohne daß dessen Aktivität wesentlich verändert wird. Diese Erfindung umfaßt deshalb auch sogenannte "funktionelle Varianten" der erfindungsgemäßen Nukleinsäuren.

Mit dem Begriff "funktionelle Varianten" sind alle DNA-Sequenzen bezeichnet, die komplementär zu einer DNA-Sequenz sind, die unter stringenten Bedingungen mit einer abgeleiteten Referenzsequenz oder Teilen davon, insbesondere der hypervariablen V(D)JC-Region, hybridisieren und eine zu dem entsprechenden erfindungsgemäßen Polypeptid ähnliche oder identische Aktivität aufweisen.

Unter "stringenten Hybridisierungsbedingungen" sind solche Bedingungen zu verstehen, bei denen eine Hybridisierung bei 60°C in 2,5 x SSC-Puffer, gefolgt von mehreren Waschschritten bei 37°C in einer geringeren Pufferkonzentration erfolgt und stabil bleibt.

Unter dem Begriff "funktionelle Varianten" im Sinne der vorliegenden Erfindung versteht man Polypeptide, die funktionell mit dem erfindungsgemäßen Polypeptiden verwandt sind, d. h. Strukturmerkmale der Polypeptide aufweisen. Beispiele funktioneller Varianten sind die entsprechenden Polypeptide, die aus anderen Organismen als der Maus, also dem Menschen, bzw., vorzugsweise aus nicht-menschlichen Säugetieren wie z. B. Affen, Schweinen und Ratten stammen. Andere Beispiele funktioneller Varianten sind Polypeptide, die durch unterschiedliche Allele des Gens, in verschiedenen Individuen oder in verschiedenen Organen eines Organismus kodiert werden. Von der vorliegenden Erfindung werden insbesondere auch funktionelle TZR-Varianten erfaßt, die das identische Epitop des MDM2-Polypeptids erkennen und eine spezifische T-Zell Antwort auslösen.

Im weiteren Sinne versteht man darunter auch Polypeptide, die eine Sequenzhomologie, insbesondere eine Sequenzidentität, von ca. 70%, vorzugsweise ca. 80%, insbesondere ca. 90%, vor allem ca. 95% zu dem Polypeptid mit der Aminosäuresequenz gemäß einer der SEQ ID Nr. 1 bis SEQ ID Nr. 6 und/oder zu anhand der Peptidsequenzen abgeleiteten DNA Sequenzen aufweisen. Darunter zählen auch Additionen, Inversionen, Substitutionen, Deletionen, Insertionen oder chemische/physikalische Modifikationen und/oder Austausche oder Teile des Polypeptids im Bereich von ca. 1-60, vorzugsweise von ca. 1-30, insbesondere von ca. 1-15, vor allem von ca. 1-5 Aminosäuren. Beispielsweise kann die erste Aminosäure Methionin fehlen, ohne daß die Funktion des Polypeptids wesentlich verändert wird.

Die Erfindung soll nun weiter anhand der beigefügten Beispiele und Figuren erläutert werden, ohne durch diese eingeschränkt zu werden. Es zeigt:
SEQ. ID Nr. 1: die alpha-Kette des murinen TZR 1tcr (1TZR_aTZR.pro),
SEQ. ID Nr. 2: die alpha-Kette des murinen MDM2 TZR (Mu_Wt_aTZR_MDM2.pro),
SEQ. ID Nr. 3: die alpha-Kette des menschlichen TZR 1bd2 (1bd2_aTCR.pro),
SEQ. ID Nr. 4: die beta-Kette des murinen TZR 1tcr (1TZR_bTZR.pro),
SEQ. ID Nr. 5: die beta-Kette des murinen MDM2 TZR (Mu_Wt_bTZR_MDM2.pro), und
SEQ. ID Nr. 6: die beta-Kette des menschlichen TZR 1bd2 (1bd2_bTCR.pro).

Figur 1 zeigt die Superposition des Protein-Rückgrats von Protein-Kristallstrukturen eines murinen H2-K^{b}-restringierten (1tcr, Garcia et al, 1998) und eines humanen HLA-A2-restringierten (1bd2, Ding et al., 1998) T-Zell-Rezeptors. Der heterodimere humane TZR ist dunkelgrau (αTZR) und hellgrau (βTZR) für die jeweiligen Ketten dargestellt; der murine TZR für die einzelnen Ketten insgesamt grau.

Figur 2 zeigt die Darstellung des Protein-Rückgrats von 1tcr mit ausschließlich denjenigen Seitenketten, die zu 1bd2 identisch sind. Die obere Graphik illustriert das Vorhandensein weniger identischer Reste im Kontaktbereich der variablen Domänen (Vα, Vβ) der beiden Ketten. Die untere Graphik ist zur oberen um die Achse in der Papierebene derart gedreht, so daß diese das zahlreiche Vorhandensein identischer Aminosäuren im Kontaktbereich der konstanten Domänen (Cα, Cβ) dokumentiert.

Figur 3 zeigt die Darstellung relevanter MDM2(81-88)-spezifischer TZR - Konstrukte, von denen im vorgestellten murinen Modell die jeweiligen Wildtyp-Ketten (Wt) des Mu Wt TZR MDM2 entsprechend der Graphik mutiert (Mut) wurden und nach retroviralem Transfer in humane T-Zellen kombiniert wurden. Die murinen variablen Domänen sind weiß, die konstanten Domänen sind grau schraffiert. Der partiell humanisierte TZR (Mu Chim TZR MDM2) besitzt eine enger gefaßte graue Schraffur in der konstanten Domäne. Unten ist ein Einzelketten-TZR abgebildet, dessen variable Domänen über einen (GlyGlyGlyGlySer)₃ - Linker verknüpft sind. Die Länge der Zylinder deuten die relevanten Aminosäurepositionen und deren sterische Größe an. Ein gelber Pfeil symbolisiert das Ausbleiben der Interaktion der Ketten aufgrund sterischer Behinderung (Mu Mutα/Wtβ TZR MDM2) bzw. des Fehlens von Interaktion (Mu Wtα/Mutβ TZR MDM2) der respektiven Aminosäurepaare.

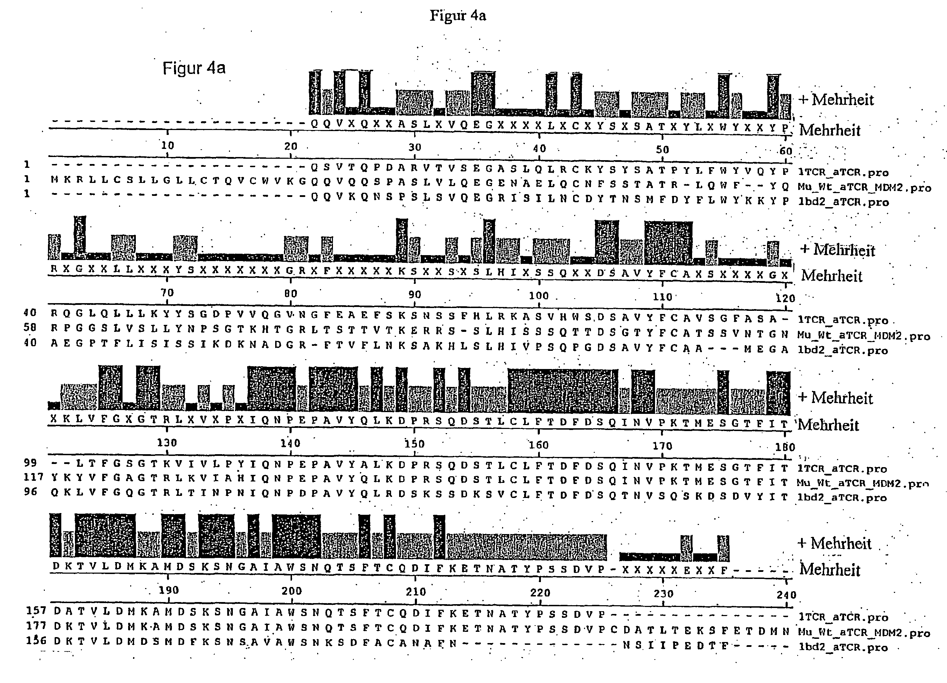

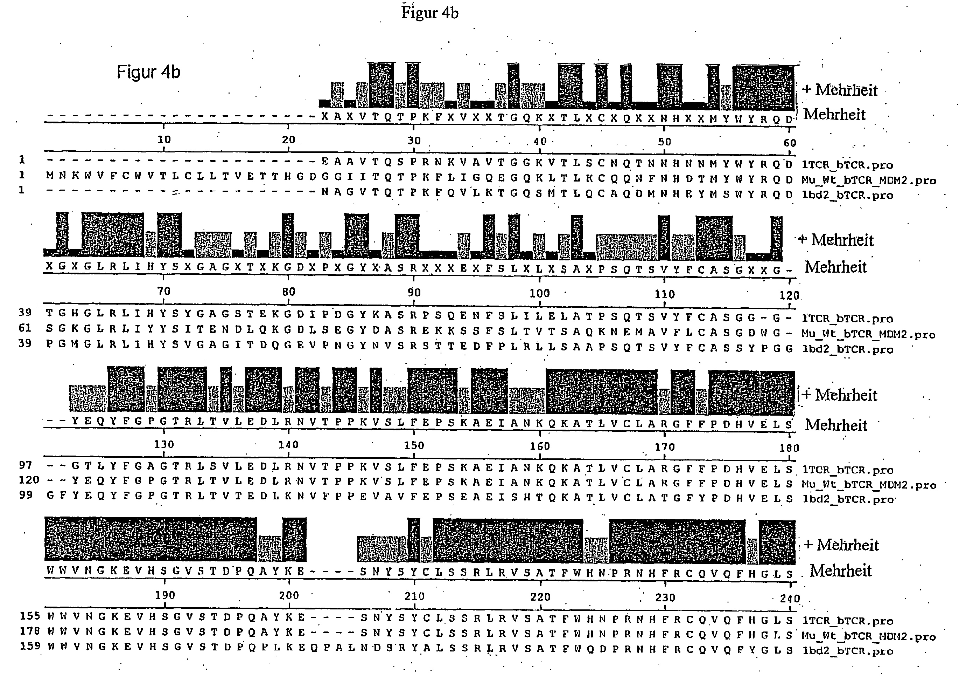

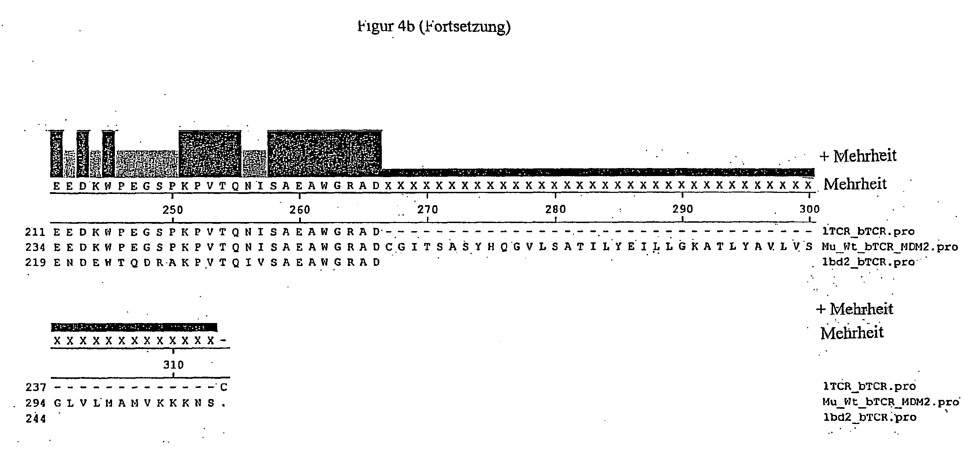

Figur 4 zeigt den Aminosäure-Sequenzvergleich des murinen TZR 1tcr (1TZR_aTZR.pro bzw. 1TZR_bTZR.pro), für den Strukturdaten vorlagen, mit den MDM2-spezifischen TZR (Mu_Wt_aTZR_MDM2.pro bzw. Mu_Wt_bTZR_MDM2.pro) und dem humanen TZR 1bd2, für den ebenfalls Strukturdaten vorliegen. Die Höhe der Balken deuten das Ausmaß der gefundenen Identität an den jeweiligen Positionen an. Aus diesem geht hervor, daß die unmittelbar die mutierten Aminosäuren umgebenden und größtenteils dazu interagierenden Aminosäuren weitgehend zwischen Mensch und Maus konserviert sind. Die Analyse der Kristall-Strukturen zeigt unwesentliche Unterschiede. Figur 4a: α-Ketten-Vergleich; Figur 4b: β-Ketten-Vergleich.

Figur 5 zeigt die Angabe des Aminosäurepaars im strukturellen Gesamtkontext von 1tcr, das dem mutierten Aminosäurepaar Gly¹⁹²/Arg²⁰⁸ des MDM2-spezifischen TZR entspricht. Die Aminosäuren liegen mittig in verdrillten, um sich gewundenen und gegenseitig aufeinander ausgerichteten β-Faltblätter beider Ketten innerhalb der konstanten Domänen. Zur Orientierung sind die relevanten CDR3-Schleifen der variablen Domänen, die das Peptid-Antigen des 1tcr erkennen, in einem abweichenden Grauton gefärbt: das betroffene Aminosäurepaar liegt weitab von der Region, die für die Bindung des MHC-Peptid-Komplexes verantwortlich ist.

Figur 6 zeigt die räumliche Darstellung des Wildtyp-Aminosäurepaars Gly¹⁷⁹/Arg¹⁹⁵ (Figur 6a) und des mutierten Aminosäurepaars Arg¹⁷⁹/Gly¹⁹⁵ (Figur 6b) des 1tcr.pdb, wie es sich für letzteres nach Strukturdaten-unterstütztem Design darstellt. Figur 6a beinhaltet diejenigen Aminosäuren der zentralen β-Faltblätter mit großen sterischen Seitenketten, die Figur 6b hingegen diejenigen Aminosäuren in einer sphärischen Region von 5 Angstroem Durchmesser um das Cα des am Bildschirm mutierten Arg¹⁷⁹ unter Wegfall derjenigen Seitenketten, die von der Kontaktregion der Ketten wegzeigen (aus Gründen der Übersicht). Es wurde eine Konformere gewählt, die keine beeinträchtigende Interaktion mit den benachbarten Seitenketten besitzt. Die gestreckte Konformation erlaubt die Ausbildung einer H-Brücke zum gegenüberliegenden Hauptketten-Sauerstoff der β-Kette nahezu analog zu den Gegebenheiten im Wildtyp (Fig 6a).

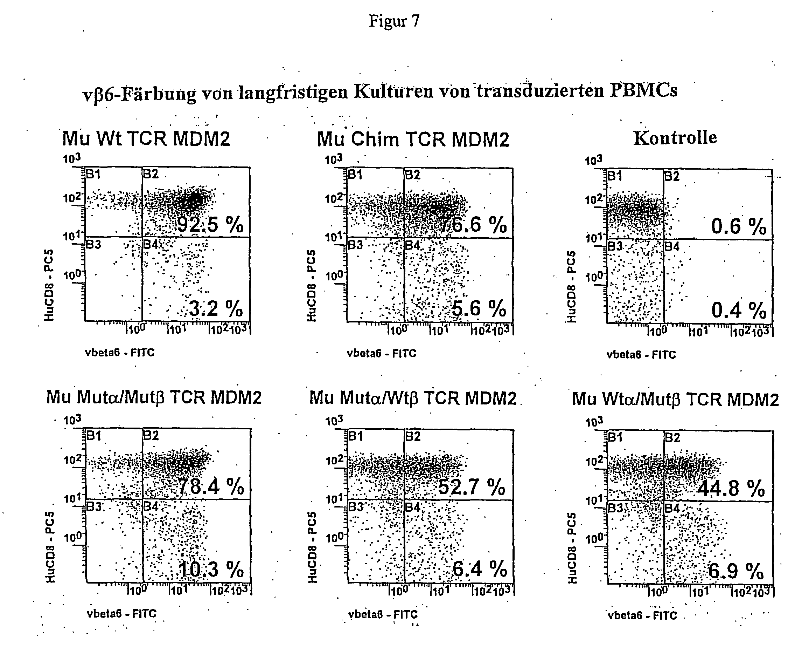

Figur 7 zeigt die FACS - Analyse der humanen transduzierten T-Zellen, die jeweils mit den unterschiedlichen Kombinationen der unter Figur 3 beschriebenen TZR-Konstrukte versehen wurden. Dargestellt ist eine 2-fach - Färbung von vβ6-FITC und CD8-PC5: nur CD8-positive, transduzierte T-Zellen zeigen die gewünschte cytotoxische Effektorfunktion. Die vβ6 - Anfärbung erlaubt den Nachweis der β-Kette, nicht der α-Kette.

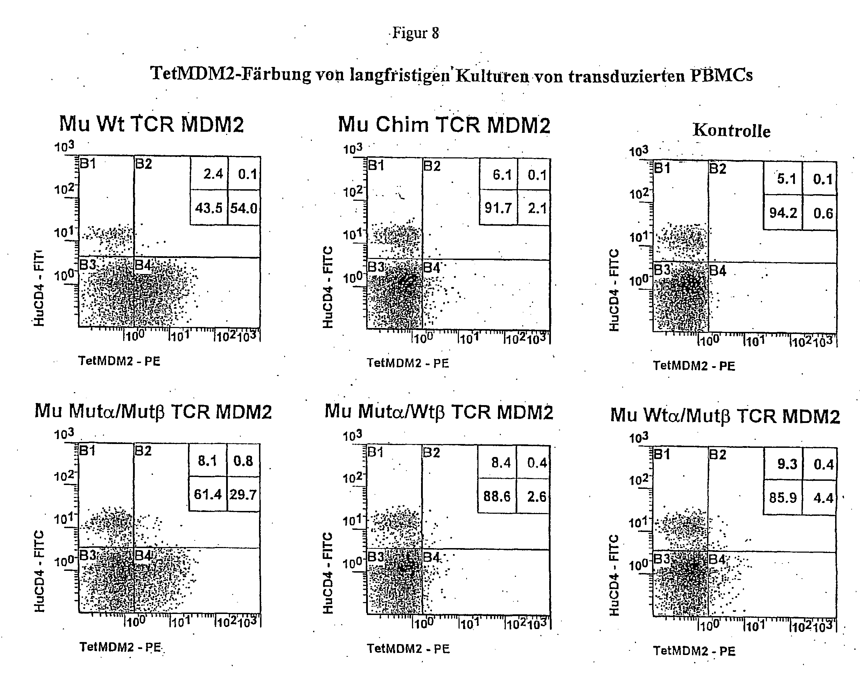

Figur 8 zeigt die FACS - Analyse der humanen transduzierten T-Zellen, die jeweils mit den unterschiedlichen Kombinationen der unter Figur 3 beschriebenen TZR-Konstrukte versehen wurden. Dargestellt ist eine 2-fach - Färbung von TetMDM2-PE und CD4-FITC: die Tetrameren-Anfärbung erlaubt den Nachweis des funktionellen, heterodimeren aßTZR, somit indirekt den Nachweis der α-Kette. Nur CD8-positive T-Zellen lassen sich anfärben, da das Tetramer im Fall moderat bis hochaffin bindender TZR nur CD8-abhängig bindet.

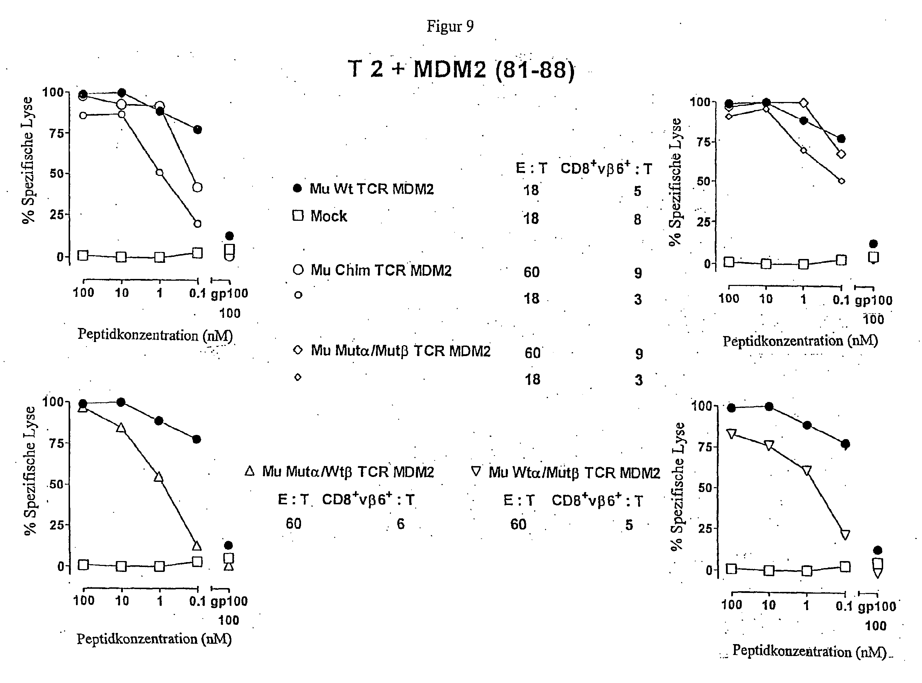

Figur 9 zeigt den Cytotoxizitäts-Test (Stanislawski & Voss et al, 2001) mit den unter Figur 3 gezeigten kombinierten Doppel-Ketten TZR: TAP-defiziente T2 wurden exogen mit den angegebenen MDM2(81-88)-Peptid-Konzentrationen beladen und auf Erkennung durch die transduzierten T-Zellen getestet: das Ausmaß der Lyse spiegelt sich in der Quantität des zellulär aufgenommen und durch Lyse freigesetzten ⁵¹Cr wieder. Als irrelevantes Peptid diente ein gp100-abgeleitetes Peptid. Es sind sowohl die "Effektor : Target" - Ratios angegeben, als auch die korrigierten CD8⁺vβ6⁺-Ratios, da dieses zwischen den unterschiedlichen Transduktions-Ansätzen schwankte.

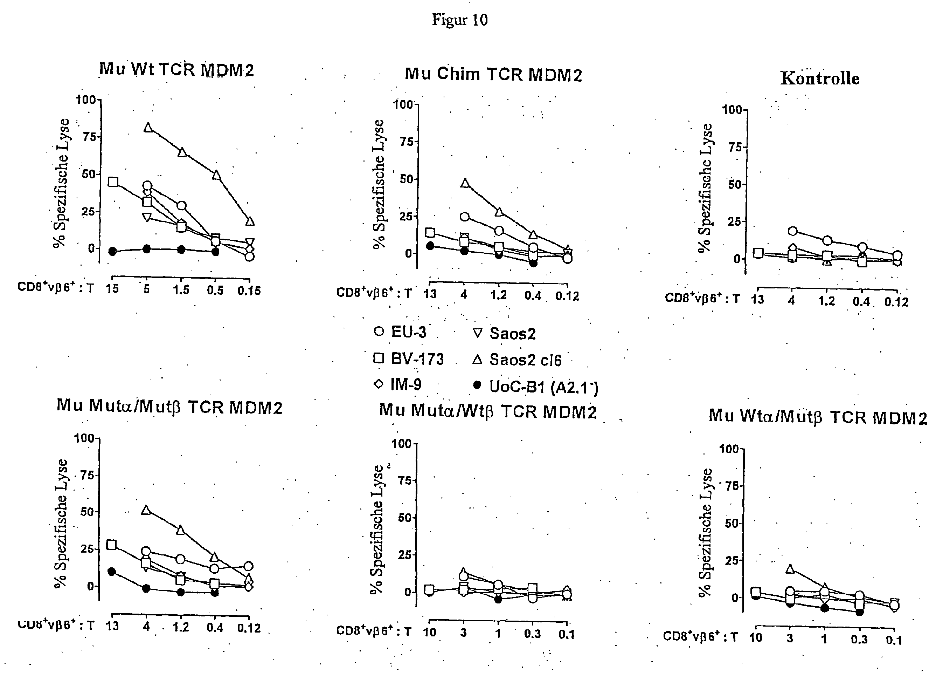

Figur 10 zeigt den Cytotoxizitäts-Test (Stanislawski & Voss et al, 2001) mit den unter Figur 3 gezeigten kombinierten Doppel-Ketten TZR: als "Targets" dienten HLA-A2-positive Leukämie-Zelllinien unterschiedlichen Ursprungs (ATCC (American Type Culture Collection, Manassas, USA), DSMZ (Deutsche Sammlung von Mikroorganismen und Zellkulturen GmbH, Braunschweig)), die MDM2 überexprimieren und das MDM2(81-88)-Peptid prozessieren. Als Negativ-Kontrolle diente die A2-negative Leukämie-Zelllinie UocB1 und die MDM2-negative Zellline Saos2. Eine MDM2-Transfektante von Saos2, Saos2 c16, wurde ebenfalls spezifisch erkannt.

Figur 11 zeigt die verschiedenen Möglichkeiten der Inversion der sterischen Verhältnisse, der Ladungen oder beidem, gemäß dem erfindungsgemäßen Verfahren.

### Beispiele

Es sollten Punktmutanten (Mut) der murinen Wildtyp-TZR (Wt) bestimmt werden, die das oben beschriebene "knob-hole"-Modell erfüllen: Kettenpaarung nur derjenigen TZR-Ketten sollte eintreten, die jeweils eine der sterisch invertierten Aminosäurepartner tragen, wohin gegen die Kombination aus Wildtyp und mutierter Kette in beiden denkbaren Orientierungen beeinträchtigt sein sollte (Figur 3). Zudem mußten die kombinierten TZR nach Einschleusen in humane T-Zellen im Hinblick auf ihre strukturelle Avidität, d.h. ihre strukturelle Integrität und in Hinblick auf ihre funktionelle Avidität, d.h. das Aufrechterhalten der Peptid-abhängigen Effektor-Funktion, getestet werden (Bullock et al., 2001). Als Modellsystem dienen die oben beschriebenen und in unserem Labor identifizierten murinen MDM2(81-88)-spezifischen T-Zell-Rezeptoren, deren Aminosäuresequenz der konstanten Domänen nahezu identisch zu dem murinen TZR 1tcr.pdb ist, für den Strukturdaten vorliegen (Figur 4a/b, Garcia et al., 1998) und der in den variablen Domänen eine hohe Homologie zu diesen aufweist. MDM2 ist ein humanes, regulatorisches Proto-Onkoprotein, das die Expression des Tumorsuppressor-Proteins p53 gegenreguliert (Stanislawski & Voss et al., 2001).

Die Art und Weise der Auswahl der Punktmutanten erfolgte nach Studium publizierter TZR-Kristallstrukturen und dem Homologievergleich muriner und humaner Sequenzen. Die Punktmutanten wurden dann in die dem Menschen ähnlichen murinen TZR eingeführt und experimentell funktionell getestet. Aus dem Homologievergleich zu humanen Sequenzen kann abgeleitet werden, daß die strukturellen Gegebenheiten in humanen TZRs in mittelbarer Umgebung der Aminosäureaustausche nahezu identisch sind und damit eine Paarung "mutierte murine Kette mit humaner Wildtyp-Kette" in der Konfiguration "αTZR - βTZR" oder "βTZR - αTZR" ebenfalls vermindert sein muß.

Die bisherigen Punktmutanten sind noch nicht absolut funktionell, behindern merkbar die Ausbildung ungewünschter Paarungen. Diese Aminosäureaustausche folgen dem allgemeinen "knob-hole" - Modell, das besagt, die Größenverhältnisse interagierender Aminosäuren zu invertieren, um eine kettenspezifische Interaktion einzuführen. Weitere Mutationen können in den bereits generierten Ketten angelegt werden, um die Spezifität weiter zu erhöhen. Dieser Aspekt ist ebenfalls vom Umfang der vorliegenden Erfindung eingeschlossen.

Die Auswahl der Punktmutanten wurde derart getroffen, daß diese zumindest für verschiedene murine TZR unterschiedlicher Peptid-Spezifität generalisierbar waren. Zudem sind diese Punktmutanten auch auf humane TZR anwendbar, da die murinen TZR in einem parallelen Projekt bei Aufrechterhaltung der Peptid-abhängigen Effektor-Funktion maximal humanisiert werden sollten, um Immunreaktionen gegen die exogenen TZR zu vermeiden. Hierfür sollten präferentiell identische Aminosäuren gegenüber homologen Aminosäuren ausgesucht werden. Ein Sequenzvergleich mehrerer muriner und humaner TZR-Sequenzen zeigte eine überaus hohe Homologie in der konstanten Domäne, wohingegen die variable Domäne nur wenig Homologien aufwies (Figur 4a/b). Es gibt zahlreiche Aminosäuren, die im Kontaktbereich der heterodimeren Ketten zwischen Mensch und Maus konserviert sind und individuell für die Aufgabenlösung verifiziert werden mußten (Figur 2). Als wesentliches Kriterium müssen die ausgewählten Aminosäuren in das "knob hole" -Modell integrierbar sein, d.h. eine Aminosäure mit einer entsprechend großen Seitenkette mußte mit einer kleinen Aminosäure der anderen Kette in Wechselwirkung treten. Hiefür spielte es *a priori* keine Rolle, in welcher der beiden Ketten sich die große Aminosäure befindet. Als große Aminosäuren kamen Tryptophan, Lysin, Arginin, Phenylalanin und Tyrosin in Betracht, als kleine insbesondere Glycin, Serin, und Alanin. Die direkt benachbarten Aminosäuren sollten sich im Fall einer potentiellen Inversion der sterischen Gegebenheiten am betreffenden Ort möglichst inert verhalten, d.h. keine ausgeprägte Interaktion, ob hydrophober oder geladener Natur, mit dem interagierenden Aminosäurepaar aufweisen.

Für das Studium muriner TZR-Strukturen wurde die Koordinaten-Datei des murinen TZR 1tcr.pdb (Garcia et al., 1998) aus der "Brookhaven Protein Data Bank" (www.resb.org/pdb) geladen und mittels der Struktur-darstellenden Software "Swiss-PDBViewer" (www.expasy.ch/spclbv) visualisiert.

Als besonders attraktiv erwies sich folgende Position: das Arginin¹⁹⁵ des βTZR aus 1tcr.pdb wies in einer nahezu gestreckten all-trans-Konformation der Seitenkette in Richtung des αTZR (Figur 5). Gegenüber der Guanidinium-Gruppe des Arg¹⁹⁵ befindet sich Gly¹⁷⁹ des αTZR in einem idealen Van der Waals - Abstand. Die Aminosäuresequenz auf Seiten der α-Kette um Gly¹⁷⁹ ist

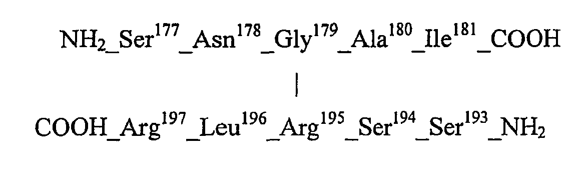

Der gegenüberliegende β-Strang der β-Kette, der das Arg¹⁹⁵ beinhaltet, ist antiparallel und ca. um 30° zum α-Ketten - β-Strang verdreht: hierdurch kommen die Seitenketten der benachbarten β-Stränge in räumliche Nähe zu dem interagierenden Aminosäurepaar. Die β-Ketten-Aminosäuresequenz an dieser Stelle ist identisch zwischen Mensch und Maus, die der α-Kette ist weitgehend homolog: Gly¹⁷⁹ ist durch die ebenfalls kleine Aminosäure Serin im Menschen ersetzt, Ile¹⁸¹ durch das homologe Valin. Die Sequenzen der auf gleicher Höhe zu den Gly¹⁷⁹/Arg¹⁹⁵ benachbarten β-Stränge beider Ketten sind über eine Länge von 5 Aminosäuren zwischen Mensch und Maus identisch und damit auch die potentiellen Interaktionspartner unmittelbar um Gly¹⁷⁹/Arg¹⁹⁵ (Figur 4a/b).

Für das Aminosäurepaar kommen allenfalls schwache hydrophobe Interaktionen benachbarter Seitenketten in Betracht. Die Kontaktfläche der Ketten an dieser Stelle ist nicht dicht gepackt: nur wenige langkettige Aminosäuren zeigen in Richtung der gegenüberliegenden Kette oder bieten großflächige hydrophobe Van der Waals-Kontakte. Dies betont um so mehr die strukturelle Bedeutung der hervorstehenden Seitenkette des Arg¹⁹⁵. Das Aminosäurepaar ist mittig in einem verdrillten β-Faltblatt der teils um sich gewundenen konstanten Domänen der beiden Ketten lokalisiert (Figur 6a/b): jedes β-Faltblatt besteht aus vier β-Strängen, von denen jeweils ein mittlerer β-Strang eine der anvisierten Aminosäuren enthält. Jedes β-Faltblatt der einzelnen Ketten bildet für sich zahlreiche H-Brücken, aber über deren Aminosäure-Seitenketten keine zu der gegenüberliegenden Kette. Ausschließlich Arg¹⁹⁵ bildet über den terminalen Stickstoff der Guanidinium-Gruppe zwei H-Brücken zum gegenüberliegenden Hauptketten-Sauerstoff und Seitenketten-Sauerstoff von Thr¹⁶⁶ der α-Kette. Es bildet eine zentrale Brücke in einer ansonsten sowohl durch wenige Salzbrücken und Dipol-Dipol-Wechselwirkungen als auch durch eine geringe Packung hydrophober Aminosäuren gekennzeichneten Kontaktregion. Am Rande dieser sich formierenden Höhle (Figur 6a) gibt es zahlreiche Kontakte zwischen den Ketten, liegen aber nicht vergleichbar günstig mitten in der Kontaktfläche der betreffenden gewundenen β-Faltblätter. Die gestreckte Konformation des Arg¹⁹⁵ als auch die geringe hydrophobe Packung ermöglichen eine Inversion der Aminosäuren, ohne die lokale Struktur wesentlich zu beeinträchtigen. Ein mutiertes Arg¹⁷⁹ des αTZR wäre ebenso in der Lage, zumindest eine H-Brücke zum Hauptketten-Sauerstoff von Ser¹⁷³ des gegenüberliegenden β-Stranges des βTZR zu bilden ohne daß das mutierte Gly¹⁹⁵ irgendeine Behinderung böte (Figur 6b). Es erfolgt eine geringfügige positive Ladungsverschiebung der Guanidinium-Gruppe von der α-Kette in Richtung der β-Kette. Die Aminosäuren müssen qualitativ nicht verändert werden, um vergleichbare sterische (d.h. ideale hydrophobe Abstände) und polare Verhältnisse (d.h. H-Brücken) zu generieren. Die zu Arg¹⁹⁵ und Gly¹⁷⁹ des 1tcr.pdb identischen Positionen im MDM2(81-88)-spezifischen TZR sind Arg²⁰⁸ und Gly¹⁹². In humanen T-Zell-Rezeptoren ist das Arginin der β-Kette konserviert, wohingegen an die Stelle des Glycins der α-Kette die ebenfalls kleine Aminosäure Serin getreten ist (1bd2; Figur 4). Eine Arbeit an mutierten bzw. trunkierten TZR belegt die Bedeutung der konstanten Domänen für die Kettenpaarung durch Coulomb-Kräfte zwischen geladenen Aminosäureresten, des Bereiches Ser¹⁸⁸-Leu²¹³ der β-Kette, in dem die von den Erfindern beschriebene Punktmutante liegt (Li et al., 1996).

Die entsprechenden Mutationen wurden mit Hilfe des "Quikchange^{™} Site-Directed Mutagenesis"-Kit (Stratagene) in die respektiven MDM2(81-88)-spezifischen TZR-Gene, die sich bereits individuell in dem retroviralen Vektor pBullet (Willemsen et al., 2000) befanden, eingeführt (Stanislawski & Voss et al., 2001).

Der adoptive Transfer in humane T-Zellen erfolgte prinzipiell wie es in Stanislawski & Voss et al (2001) beschrieben ist. Das Cotransfektionssystem (Weijtens et al., 1998), das eine Cotransfektion einzelner Plasmide mit jeweils einer Kette des heterodimeren TZR als Transgen kodierend vorsieht, ermöglichte die Kombination aller denkbaren Wildtyp und mutierten TZR-Ketten. Die Wildtyp TZR versus der in beiden Ketten mutierten TZR versus der in nur einer Kette mutierten TZR sollten strukturell über FACS-Analyse und funktionell über cytotoxische Lyse von antigenpräsentierenden Zellen (APC) als lysierbare Zielzellen analysiert werden. Hierbei dienten modellhaft die in nur einer Kette mutierten, künftig "hybride" genannte TZR als die "unerwünschten" TZR-Kettenpaare aus mutierter exogener muriner α- bzw. β-Kette und der Wildtyp endogenen humanen β- bzw. α-Kette (Figur 3), wie es hypothetisch im Fall eines adoptiven Transfers in humane T-Zellen auftreten könnte. Dies konnte angenommen werden, da sowohl das strukturelle Rückgrat als auch die Aminosäuresequenzen humaner und muriner TZR in der konstanten Domäne stark konserviert sind und potentiell interagieren können und Mutationen einen vergleichbaren Effekt auf Kettenpaarung und Antigen-Erkennung ausüben. Die in den humanen T-Zellen vorliegenden endogenen TZR störten hierbei die Analyse verschiedener muriner TZR-Kombinationen nicht, da die Mutationen der murinen Ketten allenfalls eine gleichgerichtete Wirkung auf die Kettenpaarung zu dem humanen "Pendant" hätten. Zudem ist es von Bedeutung, diese Experimente in einem experimentellen Aufbau durchzuführen, das möglichst nahe an der klinischen Applikation liegt.

Um davon auszugehen, daß alle T-Zellen das Transgen beherbergen, wurden nach Transduktion die T-Zellen über G418 (Selektionsmarker für die β-Kette, über ein IRES-Element dem Transgen nachgeschaltet), als auch über Puromycin selektiert (Selektionsmarker für die α-Kette). Somit überleben nur diejenigen T-Zellen, die sowohl die bicistronische mRNA aus βTZR-Transgen und G418-Marker als auch die bicistronische mRNA aus αTZR und Puromycin bilden. Unterschiede in der FACS-Anfärbung rühren daher nicht von unterschiedlichen Transduktionseffizienzen der humanen T-Zellen her, sondern sind Ausdruck der jeweiligen TZR-Stabilität.

Die strukturelle Avidität als Ausdruck stabiler Expression der Wildtyp als auch der mutierten TZR wurde zum einen über die Subfamilienspezifische Anfärbung der β-Kette (vbeta6-FITC; Figur 7) als auch der TZR-Spezifitäts-unterscheidenden Anfärbung mittels MDM2(81-88)-spezifischer TZR-Tetramere (Klenerman et al., 2002, Figur 8) im Wege der FACS-Analyse untersucht. Die Tetramere wurden im Labor von Dr. Pedro Romero (Universität Lausanne, Schweiz) hergestellt und für wissenschaftliche Zwecke zur Verfügung gestellt.

Es zeichnete sich bereits in der vβ6-Anfärbung ab, daß die "hybriden" TZR, Mu Mutα/Wtβ TZR MDM2 und Mu Wtβ/Mutα TZR MDM2, mindestens ebenso instabil die β-Kette exprimierten wie es von einem partiell humanisierten TZR, Mu Chim TZR MDM2, bekannt war (Figur 7). Ein weiterer Indikator einer ausgeprägten TZR-Instabilität war die nahezu vollständig fehlende Tetramer- Anfärbbarkeit (Figur 8). Die in beiden Ketten mutierten TZR, Mu Mutα/Mutβ TZR MDM2, wurden nahezu vergleichbar zu den murinen Wildtyp-Ketten, Mu Wt TZR MDM2, sowohl in der vβ6- als auch der Tetramer-Anfärbung nachgewiesen.

Die Lysiseffizienz als Maß der funktionellen Avidität wurde im Chrom-Freisetzungstest oder auch Cytotoxizitätstest gemessen. Hierzu wurde die Zelllinie T2, die nicht in der Lage ist, endogen prozessierte Peptide auf MHC-Moleküle zu laden und die Komplexe an die Zelloberfläche zu transportieren, exogen mit dem MDM2(81-88)-Peptid konzentrationsabhängig beladen und in einer Peptidtitration die halbmaximale Lyse als Maß der Erkennung gemessen (Figur 9). Die unterschiedliche vβ6- als auch CD8-Positivität der verschieden transduzierten T-Zell-Populationen wurde unter Angabe der CD8⁺vβ6⁺:T - Verhältnissen zuzüglich zu den üblichen E:T (Effektor:Target)- Verhältnissen angegeben: Unterschiede in der Lysiseffizienz sind somit ein Spiegelbild der verschieden kombinierten TZR-Konstrukte unter Korrektur der beiden T-Zell-Phänotyp-Marker als Ausdruck der prozentualen Expressionsstärke des βTZR (vβ6⁺) und der prozentualen Stärke der cytotoxischen T-Zell-Population (CD8⁺). Kongruent zu den Daten der strukturellen Avidität konnte aus den funktionellen Daten abgelesen werden, daß die "hybriden" TZR deutlich in der Lysiseffizienz im Vergleich zum Wildtyp beeinträchtigt waren und auch noch schlechter als der chimäre, partiell humanisierte TZR waren, obwohl in diesem eine komplette Domäne (Cα bzw. Cβ), und nicht nur eine Aminosäure wie in den "hybriden" TZR, ausgetauscht wurde. Der in beiden Ketten mutierte TZR, Mutα/Mutβ TZR MDM2, bewies nur leicht verschlechterte Lyseeigenschaften im Vergleich zum Wildtyp.

Diese quantitativen Unterschiede sollten nun daraufhin geprüft werden, ob es kritische halbmaximale Lysen, d.h. einen Schwellenwert gibt, ab denen Zielzellen, die endogen das betreffende Peptid präsentieren, erst erkannt werden. Hierzu wurden in einem Cytotoxizitätstest verschiedene Zielzellen überprüft (Figur 10): Saos2 dient hierbei als Negativkontrolle der MDM2-Expression, wohingegen Saos2 c16 eine MDM2-Transfektante mit positiver MDM2(81-88)-Prozessierung darstellt. Die Leukämie-Zellinie UocB1 ist HLA-A2-negativ und zeigt die MHC-Restriktion der transduzierten T-Zellen an. Die übrigen Leukämie-Zellinien EU-3, BV-173 und IM-9 sind unterschiedlichen Ursprungs (ATCC, DSMZ) und belegen die Generalisierbarkeit der MDM2(81-88)-Erkennung durch die transduzierten, humanen T-Zellen. Auch hier konnte gezeigt werden, daß der in beiden Ketten mutierte TZR vergleichbar dem Wildtyp und besser als der chimäre TZR die endogenen "Targets" erkennt. Obwohl die "hybriden" TZR die exogen beladene Zelllinie T2 bis zu 1 nM Peptid halbmaximal lysieren (Figur 9), erkennen diese die malignen Zellinien nicht: offenbar ist die MDM2(81-88)-Peptid-Präsentation unter einen kritischen Wert gefallen, unter dem dieses nicht mehr erkannt wird. Saos2 c16 wird schwach erkannt, was wahrscheinlich mit der heterologen, Promoter-getriebenen hohen Expression von MDM2 zu erklären ist.

Die Daten der strukturellen und funktionellen Avidität sind somit kongruent und belegen die Wirksamkeit der gewählten Punktmutanten in einem murinen TZR-Modell in humanen T-Zellen, um ungewünschtes "Pairing" heterodimerer "hybrider" αβTZR deutlich zu beeinträchtigen.

### Transduktion humaner peripherer Blut Lymphozyten (PBLs)

Zur Transduktion humaner peripherer T-Lymphozyten wurde ein funktionelles Derivat des pStitch-Systems (Weijtens et al., 1999) verwendet. Die zur Verpackung notwendigen retroviralen Gene werden über individuelle Plasmide im Wege einer Cotransfektion der Verpackungszellinie 293T kodiert (Soneoka et al., 1995): pHit60 kodiert für die *gag-pol -* Struktur- und Polymerase - Gene aus dem Moloney murinen Leukämie Virus (MoMuLV), pColt-Galv für das *env -* Hüllprotein des "gibbon ape leukemia virus", das imstande ist, an den Na⁺-/Phosphat-Synporter Pit humaner Zellen zu binden und damit letztere zu transduzieren. Die chimären Viruspartikel besitzen somit einen amphotropen Pseudotyp und können verschiedene Säugerzellen, außer Maus, transduzieren.

### Transfektion der Verpackungszelllinie 293T

Die isolierten bakteriellen Klone der in das pStitch-Derivat klonierten T-Zell-Rezeptor-Gene wurden über Plasmid-Präparationen, die eine Entfernung residueller Endotoxine versprechen (Qiagen, Produkt 12362), gereinigt und zu 1 µg/µl eingestellt. Die DNS wurde über die Calciumphosphat-Präzipitation transient in die Verpackungszelllinie 293T eingeführt (GiboBRL-Life Technologies, Produkt 18306-019). Hierbei werden im Rahmen der modifizierten T-Zell-Rezeptoren αTZR und βTZR bis zu 80 µg DNS eingesetzt:
20 µg αTZR - Konstrukt
20 µg βTZR - Konstrukt
20 µg pColt-Galv
20 µg pHit 60

Im Fall von Einzelketten-TZRs werden 60 µg DNS eingesetzt. 293T wurde in einem modifizierten DMEM-Medium (DMEM/H) kultiviert:
DMEM, 4,5% Glukose (BioWhittaker)
10% hitzeinaktiviertes FKS
2mM Glutamin
1x Penicillin/Streptomycin
1x nicht-essentielle Aminosäuren
25 mM HEPES

Am Vortag der Transfektion wurden die Zellen 293T auf 0,9*10⁶ Zellen pro T25-Flasche und Transfektionsansatz in 5ml DMEM/H ausgesät. 4 h vor Transfektion wurde das Medium gegen frisches, auf Raumtemperatur (RT) erwärmtes DMEM-H (3m1) ersetzt. Die Transfektion erfolgte laut Vorschrift des kommerziellen Protokolls (Invitrogen). 1 ml des Transfektionsansatzes wurde zu der jeweiligen Flasche unter vorsichtigem Hinzutropfen pipettiert. Das DNS-Ca₃(PO₄)₂-Präzipität sollte fein verteilt sich auf den adhärenten Zellen niederlegen.

Am darauffolgenden Morgen wurde das Medium gegen frisches, auf RT erwärmtes DMEM/H ausgetauscht. 6 h später erfolgte die Kokultivierung mit den aktivierten PBLs.

### Transduktion der aktivierten PBLs - Aktivierung peripherer Blut-Lymphozyten (PBLs)

3 Tage vor der angesetzten Kokultivierung wurden fikollisierte PBLs zu 2*10⁶ Zellen/ml in huRPMI-P jeweils in 2 ml einer 24 Well-Platte (Zellgewebe-behandelte Oberflächen) ausgesät. Die Aktivierung erfolgte über den kreuz-vernetzenden Antikörper OKT-3 (Orthoclone-Diagnostics) zu 20 ng/ml.
huRPMI-P:
RPMI 1640 (2mM Glutamin) ohne Phosphat (Life Tec., 11877-032) 10 % humanes, hitzeinaktiviertes AB-Serum (HLA-A2.1 seropositiv) 25 mMHEPES
1 x Penicillin/Streptomycin (Life Tec.)

Die Platten wurden in einem Brutschrank bei 37°C und 5% CO2 inkubiert.

### Kokultivierung

Zur Kokultivierung wurden die aktivierten PBLs aus den jeweiligen Vertiefungen einer 24-Well-Platte vereinigt und gezählt. Adhärente Monozyten wurden verworfen. Die Zellen werden abzentrifugiert (1500 rpm, 5 min, RT) und in einer Konzentration von 2,5* 10⁶ Zellen/ml in frischem huRPMI-P aufgenommen und in den Brutschrank zurückgestellt. Das Medium wurde zuvor zu 400 U/ml IL-2 (Chiron) und 5 µg/ml Polybren (Sigma) eingestellt.

Jeder Transfektionsansatz wurde 6h nach Mediumwechsel nacheinander trypsiniert: hierzu wurde jede T25 mit 3 ml HBSS (Life Technologies) gewaschen, mit 1 ml Trypsin-EDTA (Life Technologies) für maximal 5 Minuten inkubiert, die gelösten Zellen quantitativ aufgenommen und in 4ml vorgelegtes huRPMI-P (RT) unter Rühren getropft. Die 293T Zellen werden mit 2500 rad bestrahlt. Diese werden abzentrifugiert (1500 rpm, 5 min, RT) und in 4 ml frisches, eingestelltes huRPMI-P, mit 400U/ml IL-2 und 5µg/ml Polybren supplementiert, resuspendiert. Zu dem Ansatz wurde 1 ml der eingestellten PBLs gegeben und der Ansatz (0,5* 10⁶ PBLs/ml) für drei Tage im Brutschrank (37°C, 5% CO2) inkubiert.

Am Tag 3 nach Kokultivierung wurden die suspendierten PBLs abgenommen und in frischem, mit 40 U/ml IL-2 (Chiron) und 2,5 µl CD3/CD28-Beads supplementiertem Medium huRPMI-P zu 1*10⁶ Zellen/ml resuspendiert. 3 Tage später erfolgte ein neuerlicher Split in frisches Medium. In diesen 7 Tagen wurde maximal expandiert bis zum Übergang auf T75-Flaschen. Diese Zellen konnten direkt in einer immunologischen Anfärbung (FACS-Analyse) oder in einem klassischen ⁵¹Chrom-Freisetzungstest eingesetzt werden.

### Beispiele der FACS-Analyse

Die oben beschriebenen Konstrukte wurden nach retroviraler Transduktion im "fluorescence activated cell sorting" (FACS) analysiert. Hierzu wurden 0,25*10⁶ Zellen sättigend mit Fluorophor-markierten Antikörpern gefärbt: die heterolog exprimierte mutierte β-Kette wurde mit anti-vβ6-FITC (BD) nachgewiesen; die Gesamtheit der T-Zellen über den Marker anti-CD3-PC5 (Coulter-Beckman). Als Negativ-Kontrolle diente eine mit dem leeren pStitch-Derivat transduzierte Probe. Die Expression konnte in mehreren Donoren HLA-A2 - positiver T-Zellen reproduziert werden. Für die Tetramer-Anfärbung wurden 5 µl einer 0,28 mg/ml Vorratslösung für 45 min bei 8°C verwendet.

### Cytolytische Aktivität der transduzierten T-Zellen

Die transduzierten T-Zellen wurden in einem klassischen ⁵¹Chrom-Freisetzungstest auf ihre zytotoxische Spezifität hin überprüft. In diesem System wurden Zielzellen radioaktiv durch Inkorporation von ⁵¹Chrom markiert. Erkannten die retroviral modifizierten Effektorzellen die Zielzelle peptidspezifisch, wurden letztere durch die Effektorfunktionen der T-Zelle in die Apoptose getrieben und durch Lyse getötet. Das Ausmaß des freigesetzten Chrom-Nuklids trifft eine Aussage über die Effektivität der Zell-Erkennung und Lyse. Die Effektivität wurde über einen weiten Bereich des Verhältnisses eingesetzter Effektor-Zellen zu Zielzellen (E:T) getestet. Als Referenz diente ein muriner MDM2-81-88 peptidspezifischer T-Zell-Klon, aus dem T-Zell-Rezeptor-Gene isoliert wurden. Als Zielzellen dienten:
T2: humane TAP-defiziente Zelllinie die exogen mit beliebigem Peptid zu beladen war. Das spezifische mutierte Peptid war MDM2-81-88, ein irrelevantes Kontroll-Peptid entstammte dem Influenza-Matrixprotein FluM1.
Saos-2/6: Transfektante der humanen Osteosarcoma Saos-2-Zelllinie, die MDM2 heterolog exprimiert und endogen prozessiert.
UocB11, EU-3: prä-B-Zell-Leukämie
IM-9: Plasmocytom
BV173: prä-B-Zell-Leukämie

## Patentansprüche

1. Verfahren zur Herstellung eines rekombinanten heterodimeren spezifischen Wildtyp- oder chimären T-Zell Rezeptors (TZR), so dass bevorzugt die von außen zugeführten TZR-Ketten paaren und nicht gemischte Pärchen mit den endogenen Ketten der T-Zellen ausbilden, ohne dabei deren Funktionalität und Stabilität zu beeinträchtigen, wobei der TZR eine erste Kette und eine zweite Kette enthält, die miteinander an mindestens einer Oberfläche ihrer Aminosäuren wechselwirken, wobei die mindestens eine Aminosäuren-Oberfläche einer rationalen Mutagenese unterzogen wird, so dass die mindestens eine Aminosäuren-Oberfläche der ersten Kette oder die Aminosäuren-Oberfläche der zweiten Kette eine räumlich hervorstehende chemische Gruppe umfaßt, die mit einer räumlich zurückgesetzten chemischen Gruppe auf der mindestens einen Oberfläche der im TZR entsprechend gepaarten ersten Kette oder zweiten Kette wechselwirkt, umfassend die Schritte von:
(a) zu Verfugung stellen der DNA-Moleküle, die die kodierenden Bereiche für die mindestens eine zu mutierende Aminosäuren-Oberfläche der ersten Kette oder zweiten Kette umfassen, in einem gemeinsamen oder getrennten Mutagenese-Vektorsystem(en),
(b) Mutagenese der DNA-Moleküle auf an sich bekannte Weise, wobei die für die mindestens eine Aminosäuren-Oberfläche kodierende Nukleinsäuresequenz im Vergleich zu der Ausgangssequenz so verändert wird, dass
in die mindestens eine Aminosäuren-Oberfläche der ersten Kette oder die mindestens eine Aminosäuren-Oberfläche der zweiten Kette gegenüber der ursprünglichen Aminosäuren-Oberfläche eine räumlich hervorstehende chemische Gruppe eingeführt wird, und
in die entsprechend in Wechselwirkung stehende mindestens eine Aminosäure-Oberfläche der zweiten Kette oder der ersten Kette gegenüber der ursprünglichen Aminosäuren-Oberfläche(n) eine räumlich zurückgesetzte chemische Gruppe eingeführt wird, wodurch einzelne Mutanten-Fragmente hergestellt werden,
wobei die nach der Mutagenese der DNA-Moleküle eingeführte Aminosäure, die gegenüber der ursprünglichen Aminosäuren-Oberfläche eine räumlich zurückgesetzte Gruppe einführt, ausgewählt ist aus Glyzin, Serin, Threonin, Valin und Alanin und wobei die nach der Mutagenese der DNA-Moleküle eingeführte Aminosäure, die gegenüber der ursprünglichen Aminosäuren-Oberfläche eine räumlich hervorstehende Gruppe einführt, ausgewählt ist aus Glutamin Glutaminsäure alpha-Methylvalin Histidin Hydroxylysin. Tryptophan Lysin, Arginin, Phenylalanin und Tyrosin, und
c) Translation von mindestens zwei der einzelnen Mutanten-Fragmente aus Schritt b) in vitro oder in isolierten Zellen, so dass die Paarung des heterodimeren, mindestens an einer Aminosäure-Oberfläche mutierten spezifischen ersten-Kette/zweite-Kette TZR selektiv gefördert wird, und
d) Präsentation des heterodimeren erste-Kette/zweite-Kette TZR durch eine T-Zelle.

2. Verfahren nach Anspruch 1, wobei Schritt c) durch folgende Schritte ersetzt wird:
(c') gegebenenfalls Umklonierung der Mutanten-Fragmente in geeignete Transfektions-Vektorsysteme,
(c") Transfektion oder Cotransfektion oder Transduktion mindestens zwei der Mutanten-Fragmente in eine Mutanten TZR-defiziente T-Zelle, und
(c''') Expression des heterodimeren erste-Kette/zweite-Kette TZR in einer rekombinanten T-Zelle.

3. Verfahren nach Anspruch 1, wobei Schritt c) durch folgende Schritte ersetzt wird:
c') *In vitro*-Translation oder *in vivo*-Translation in isolierten Zellen von mindestens zwei der einzelnen Mutanten-Fragmente aus Schritt b) und gegebenenfalls anschließende Isolierung und/oder Reinigung der translatierten Mutanten-Fragmente, so dass die Paarung des heterodimeren, mindestens an einer Aminosäure-Oberfläche mutierten spezifischen ersten-Kette/zweite-Kette TZR selektiv gefördert wird, und
c") Einbringung der mutierten spezifischen ersten-Kette/zweite-Kette TZR in eine T-Zelle.

4. Verfahren nach Anspruch 3, wobei die *in vivo* Translation in einer Wirtszelle erfolgt.

5. Verfahren nach Anspruch 3 oder 4, wobei die Einbringung durch Liposomen-Transfer erfolgt.

6. Verfahren nach einem der Ansprüche 1 bis 5, wobei es sich bei dem TZR um einen alpha/beta TZR, gamma/delta TZR, einen humanisierten oder partiell humanisierten TZR, einen mit zusätzlichen (funktionellen) Domänen versehenen TZR, einen mit alternativen Domänen versehenen TZR, z.B. mit einer anderen Transmembran-Domäne als Membrananker versehenen TZR handelt.

7. Verfahren nach einem der Ansprüche 1 bis 6, wobei die nach der Mutagenese der DNA-Moleküle eingeführte Aminosäure weiter geeignet chemisch modifiziert wird, um dadurch eine räumlich hervorstehende Gruppe oder eine räumlich zurückgesetzte Gruppe einzuführen.

8. Verfahren nach einem der Ansprüche 1 bis 6, wobei die nach der Mutagenese der DNA-Moleküle eingeführte Aminosäure die räumlich hervorstehende Gruppe oder die räumlich zurückgesetzte Gruppe direkt zur Verfügung stellt.

9. Verfahren nach einem der Ansprüche 1 bis 8, wobei die durch die Mutagenese der DNA-Moleküle eingeführten Aminosäuren so gewählt sind, dass ein wechselseitiger Austausch der Aminosäuren der an den Aminosäure-Oberflächen der wechselwirkenden Ketten im entsprechend gepaarten TZR erreicht wird.

10. Verfahren nach einem der Ansprüche 1 bis 9, wobei mindestens zwei Aminosäure-Oberflächen einer TZR-Kette gleichzeitig einer Mutagenese unterzogen werden.

11. Verfahren nach einem der Ansprüche 1 bis 10, wobei die zu mutierenden Domänen der TZR-Ketten ausgewählt sind aus Säugetier, insbesondere menschlichen und/oder Maus-Domänen.

12. Verfahren nach einem der Ansprüche 1 bis 11, wobei die rationale Mutagenese der TZR-Ketten gleichzeitig zu einer Humanisierung des TZR führt.

13. Verfahren nach einem der Ansprüche 1 bis 12, wobei als Transfektionssystem ein retroviraler Vektor, insbesondere pBullet verwendet wird.

14. Verfahren zur Herstellung einer pharmazeutischen Zusammensetzung, umfassend ein Verfahren nach einem der Ansprüche 1 bis 13 und Mischen des hergestellten heterodimeren spezifischen Wildtyp- oder chimären T-Zell Rezeptors mit einem pharmazeutisch akzeptablen Träger.

## Claims

1. Method for producing a recombinant heterodimeric specific wild type- or chimeric T-cell receptor (TCR) in a manner that preferably the externally provided TCR-chains do pair and do not form mixed pairs with the endogenous chains of the T-cells, without that their functionality and stability is impaired, wherein said TCR contains a first chain and a second chain that interact one with the other at at least one surface of their amino acids, whereby the said at least one amino acid-surface is subjected to a rational mutagenesis in a manner that said at least one amino acid-surface of the first chain or said amino acid-surface of the second chain comprises a sterically projecting chemical group interacting with a sterically recessed group on the at least one surface of the first chain or second chain as correspondingly paired in the TCR, comprising the steps of:
(a) providing of the DNA-molecules that comprise the coding regions for the at least one amino acid-surface to be mutated of the first chain the or second chain, in (a) combined or separate mutagenesis-vector system(s),
(b) mutagenesis of the DNA-molecules in a manner known as such, wherein the nucleic acid sequence encoding for the at least one amino acid-surface is modified compared with the initial sequence in such a manner that
into the at least one amino acid-surface of the first chain or the at least one amino acid-surface of the second chain, a sterically projecting group is introduced compared with the initial amino acid-surface of the second chain, and
into the respective interacting at least one amino acid-surface of the second chain or the first chain, a sterically recessed group is introduced, compared to the initial amino acid-surface(s), whereby individual mutated fragments are produced,
wherein the amino acid as introduced after the mutagenesis of the DNA-molecules that introduces a sterically recessed group compared with the initial amino acid-surface is selected from glycine, serine, threonine, valine, and alanine, and wherein the amino acid as introduced after the mutagenesis of the DNA-molecules that introduces the sterically projecting group of the initial amino acid-surface compared with the initial amino acid-surface is selected from glutamine, glutamic acid, alpha-methylvaline, histidine, hydroxylysine, tryptophan, lysine, arginine, phenylalanine, and tyrosine, and
c) translation of at least two of the individual mutant-fragments from step b) in vitro or in isolated cells, in a manner that pairing of the heterodimeric first-chain/second-chain TCR being mutated at the at least one amino acid-surface is selectively facilitated, and
d) presentation of said heterodimeric first-chain/second-chain TCR by a T-cell.

2. Method according to claim 1, wherein step c) is replaced by the following steps:
(c') optionally, sub-cloning of the mutant-fragments into suitable transfection-vector systems,
(c'') transfection or co-transfection or transduction of at least two of the mutated fragments into a mutant TCR-deficient T-cell, and
(c''') expression of the heterodimeric first-chain/second-chain TCR in a recombinant T-cell.

3. Method according to claim 1, wherein step c) is replaced by the following steps:
c') *In vitro*-translation or *in vivo*-translation in isolated cells of at least two of the individual mutant-fragments from step b) and, optionally, subsequent isolation and/or purification of the translated mutant-fragments,
in a manner that the pairing of the heterodimeric specific first-chain/second-chain TCR being mutated at the at least one amino acid-surface is selectively facilitated, and
c'') introduction of the mutated specific first-chain/second-chain TCR into a T-cell.

4. Method according to claim 3, wherein *the in vivo* translation takes place in a host cell.

5. Method according to claim 3 or 4, wherein the introduction takes place through liposome-transfer.

6. Method according to any of claims 1 to 5, wherein said TCR is an alpha/beta TCR, gamma/delta TCR, a humanized or partially humanized TCR, a TCR provided with additional (functional) domains TCR, a TCR provided with alternative domains, e.g. a TCR provided with a different transmembrane-domain as a membrane anchor.

7. Method according to any of claims 1 to 6, wherein said amino acid as introduced after the mutagenesis of the DNA-molecules furthermore is suitably chemically modified, in order to thereby introduce a sterically projecting group or a sterically recessed group.

8. Method according to any of claims 1 to 6, wherein the amino acid as introduced after the mutagenesis of the DNA-molecules directly provides said sterically projecting group or said sterically recessed group.

9. Method according to any of claims 1 to 8, wherein the amino acids as introduced through the mutagenesis of the DNA-molecules are selected in a manner that a reciprocal exchange of the amino acids at the amino acid-surfaces of the interacting chains in the correspondingly paired TCR is achieved.

10. Method according to any of claims 1 to 9, wherein at least two amino acid-surfaces of a TCR-chain are simultaneously subjected to a mutagenesis.

11. Method according to any of claims 1 to 10, wherein the domains of the TCR-chains to be mutated are selected from mammalian, in particular human and/or mouse-domains.

12. Method according to any of claims 1 to 11, wherein the rational mutagenesis of the TCR-chains leads to a simultaneous humanization of the TCR.

13. Method according to any of claims 1 to 12, wherein a retroviral vector, in particular pBullet, is used as a transfection system.

14. Method for producing a pharmaceutical composition, comprising a method according to any of claims 1 to 13, and mixing of said heterodimeric specific wildtype or chimeric T-cell receptor as produced with a pharmaceutically acceptable carrier.

## Revendications

1. Procédé de préparation d'un récepteur de lymphocytes (TZR) de type sauvage ou chimère spécifique hétérodimère recombinant, de sorte que de préférence les chaînes de TZR provenant de l'extérieur s'apparient et ne forment pas de paires mixtes avec les chaînes endogènes des lymphocytes, sans diminuer leur fonctionnalité et leur stabilité, le TZR contenant une première chaîne et une seconde chaîne, lesquelles interagissent ensemble au niveau d'au moins une surface de leurs acides aminés, au moins une surface des acides aminés étant soumise à une mutagenèse rationnelle, de sorte qu'au moins une surface des acides aminés de la première chaîne ou la surface des acides aminés de la seconde chaîne comprend un groupe chimique sortant, lequel groupe interagit avec un groupe chimique rentrant sur la au moins une surface de la première chaîne ou seconde chaîne appariée correspondante du TZR, comprenant les étapes consistant à :
(a) rendre les molécules d'ADN disponibles, lesquelles comprennent les zones codantes pour la au moins une surface des acides aminés à muter de la première chaîne ou la seconde chaîne, dans un (des) système(s) de vecteur de mutagenèse commun(s) ou séparé(s),
(b) effectuer la mutagenèse des molécules d'ADN selon un procédé connu en soi, la séquence d'acides nucléiques codant au moins une surface d'acides aminés est modifiée comparativement à la séquence de sortie de sorte que
un groupe chimique sortant est introduit dans la au moins une surface d'acides aminés de la première chaîne ou la au moins une surface d'acides aminés de la seconde chaîne par rapport à la surface d'acides aminés d'origine, et
un groupe chimique rentrant est introduit dans la au moins une surface d'acides aminés correspondante en interaction de la seconde chaîne ou la première chaîne par rapport à la (aux) surface(s) d'acides aminés d'origine, moyennant quoi des fragments de mutants individuels peuvent être préparés,
les acides aminés introduits dans les molécules d'ADN après la mutagenèse, lesquels acides aminés présentant un groupe rentrant par rapport à la surface d'acides aminés d'origine, sont choisis parmi la glycine, la sérine, la thréonine, la valine et l'alanine et les acides aminés introduits dans les molécules d'ADN après la mutagenèse, lesquels acides aminés présentant un groupe sortant par rapport à la surface d'acides aminés d'origine, sont choisis parmi la glutamine, l'acide glutaminique, l'alpha-méthylvaline, l'histidine, l'hydroxylysine, le tryptophane, la lysine, l'arginine, la phénylalanine et la tyrosine, et
(c) effectuer une translation d'au moins deux des fragments de mutants individuels de l'étape (b) in vitro ou dans des cellules isolées, de sorte que l'appariement du TZR à la première chaîne/seconde chaîne spécifique mutant hétérodimère de la surface d'acides aminés se produit sélectivement, et
(d) présenter le TZR hétérodimère à première chaîne/seconde chaîne via un lymphocyte.

2. Procédé selon la revendication 1, dans lequel l'étape
(c) est remplacée par les étapes suivantes consistant à : (c') le cas échéant transcloner les fragments de mutants dans des systèmes de vecteur de transfection appropriés,
(c'') transfecter ou cotransfecter ou effectuer la transduction d'au moins deux des fragments de mutants dans un lymphocyte mutant déficient en TZR, et
(c''') exprimer le TZR hétérodimère à première chaîne/seconde chaîne dans un lymphocyte recombinant.

3. Procédé selon la revendication 1, dans lequel l'étape (c) est remplacée par les étapes suivantes consistant à :
(c') translater in vitro ou translater in vivo dans des cellules isolées au moins deux des fragments de mutants individuels de l'étape (b) et le cas échéant isolement ultérieur et/ou purification des fragments de mutants translatés,
de sorte que l'appariement du TZR à première chaîne/seconde chaîne spécifique mutant hétérodimère au moins à la surface d'acides aminés s'effectue de manière sélective, et
(c'') déposer le TZR à première chaîne/seconde chaîne spécifique mutant dans un lymphocyte.

4. Procédé selon la revendication 3, dans lequel la translation in vivo est effectuée dans une cellule hôte.

5. Procédé selon la revendication 3 ou 4, dans lequel l'introduction est effectuée par un transfert de liposomes.

6. Procédé selon l'une quelconque des revendications 1 à 5, dans lequel le TZR est un TZR alpha/bêta, un TZR gamma/delta, un TZR humain ou partiellement humain, un TZR doté de domaines (fonctionnels) supplémentaires, un TZR doté de domaines en variante, par exemple doté d'autres domaines transmembranaires que l'ancre de membrane.

7. Procédé selon l'une quelconque des revendications 1 à 6, dans lequel les acides aminés introduits dans les molécules d'ADN après la mutagenèse sont modifiés de manière chimiquement appropriée, afin d'introduire ainsi un groupe sortant ou un groupe rentrant.

8. Procédé selon l'une quelconque des revendications 1 à 6, dans lequel les acides aminés introduits dans les molécules d'ADN après la mutagenèse rendent le groupe sortant ou le groupe rentrant directement disponible.

9. Procédé selon l'une quelconque des revendications 1 à 8, dans lequel les acides aminés introduits dans les molécules d'ADN après la mutagenèse sont choisis de sorte qu'un échange mutuel des acides aminés des surfaces d'acides aminés des chaînes en interaction est atteint dans le TZR apparié correspondant.

10. Procédé selon l'une quelconque des revendications 1 à 9, dans lequel au moins deux surfaces d'acides aminés d'une chaîne de TZR sont soumises simultanément à une mutagenèse.

11. Procédé selon l'une quelconque des revendications 1 à 10, dans lequel les domaines à muter des chaînes de TZR sont choisis parmi les mammifères, en particulier les domaines humains et/ou murins.

12. Procédé selon l'une quelconque des revendications 1 à 11, dans lequel la mutagenèse rationnelle des chaînes de TZR aboutit simultanément à une humanisation du TZR.

13. Procédé selon l'une quelconque des revendications 1 à 12, dans lequel un vecteur rétroviral, en particulier pBullet est utilisé en tant que système de transfection.

14. Procédé de préparation d'une composition pharmaceutique, comprenant un procédé selon l'une quelconque des revendications 1 à 13 et des mélanges du récepteur de lymphocytes de type sauvage ou chimère spécifique hétérodimère préparé avec un véhicule pharmaceutiquement acceptable.
